(19)

**Europäisches Patentamt**
**European Patent Office**
**Office européen des brevets**

(11)     **EP 4 120 277 B1**

(12)                   **EUROPEAN PATENT SPECIFICATION**

(45) Date of publication and mention
of the grant of the patent:
**22.04.2026   Bulletin 2026/17**

(21) Application number: **22185188.4**

(22) Date of filing: **15.07.2022**

(51) International Patent Classification (IPC):
**G16B 15/30** *(2019.01)*     **G16B 40/20** *(2019.01)*

(52) Cooperative Patent Classification (CPC):
**G16B 15/30; G16B 40/20**

(54) **DRUG RANKING METHOD AND SYSTEM, COMPARISON METHOD FOR DRUG RANKING METHOD, CORRECTION METHOD FOR DRUG RANKING METHOD**

ARZNEIMITTELKLASSIFIZIERUNGSVERFAHREN UND -SYSTEM, VERGLEICHSVERFAHREN FÜR ARZNEIMITTELKLASSIFIZIERUNGSVERFAHREN, KORREKTURVERFAHREN FÜR ARZNEIMITTELKLASSIFIZIERUNGSVERFAHREN

PROCÉDÉ ET SYSTÈME DE CLASSEMENT DE MÉDICAMENTS, PROCÉDÉ DE COMPARAISON POUR PROCÉDÉ DE CLASSEMENT DE MÉDICAMENTS, PROCÉDÉ DE CORRECTION POUR PROCÉDÉ DE CLASSEMENT DE MÉDICAMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.07.2021   US 202163222438 P**

(43) Date of publication of application:
**18.01.2023   Bulletin 2023/03**

(73) Proprietor: **National Tsing Hua University**
**Hsinchu City 300 (TW)**

(72) Inventors:
• **YANG, Lee-Wei**
**300 Hsinchu City (TW)**
• **TSAI, Kun-Lin**
**300 Hsinchu City (TW)**
• **LIN, Tzu-Kuan**
**300 Hsinchu City (TW)**

(74) Representative: **Viering, Jentschura & Partner mbB**
**Patent- und Rechtsanwälte**
**Am Brauhaus 8**
**01099 Dresden (DE)**

(56) References cited:
• **TSAI KUN-LIN ET AL: "DRDOCK: A Drug Repurposing platform integrating automated docking, simulations and a log-odds-based drug ranking scheme", BIORXIV, 2 February 2021 (2021-02-02), XP093270036, Retrieved from the Internet <URL:https://www.biorxiv.org/content/10.1101/2021.01.31.429052v1.full.pdf> [retrieved on 20250414], DOI: 10.1101/2021.01.31.429052**
• **TSAI KUN-LIN ET AL: "DRDOCK: A Drug Repurposing platform integrating automated docking, simulations and a log-odds-based drug ranking scheme", BIORXIV, 2 June 2021 (2021-06-02), XP093001651, Retrieved from the Internet <URL:https://www.biorxiv.org/content/10.1101/2021.01.31.429052v3.full> [retrieved on 20221124], DOI: 10.1101/2021.01.31.429052**
• **BOLELLI KAYHAN ET AL: "Fenoterol and dobutamine as SARS-CoV-2 main protease inhibitors: A virtual screening study", JOURNAL OF MOLECULAR STRUCTURE, ELSEVIER AMSTERDAM, NL, vol. 1228, 13 October 2020 (2020-10-13), XP086445251, ISSN: 0022-2860, [retrieved on 20201013], DOI: 10.1016/J.MOLSTRUC.2020.129449**

**Description**

**BACKGROUND**

**Technical Field**

The instant disclosure relates to a ranking method and a ranking system to rank a plurality of drugs.

**Related Art**

Drug repurposing, where drugs originally approved to treat a disease are reused to treat other diseases, have received escalating attention, especially in pandemic years. Structure-based drug design, which integrates small molecule docking and artificial intelligence, has shown its obvious importance in streamlining new drug development as well as drug repurposing. In order to perform a sophisticated and fully automated drug screening of multiple drugs (e.g., all FDA-approved drugs), accurate drug ranking methods are to be developed.

The publication "DRDOCK: A Drug Repurposing platform integrating automated docking, simulations and a log-odds-based drug ranking scheme" by Kun-Lin Tsai (bioRxiv, 2) relates to a web-based drug repurposing platform that integrates automated molecular docking, molecular dynamic (MD) simulations, and a log-odds-based drug ranking scheme. 2016 FDA-approved drugs are screened against user-submitted single-chain protein targets. The platform ranks drugs based on docking poses and allows users to select poses for further MD-based binding affinity evaluation. This publication does not specifically define which active site residues of a target protein are used to calculate distance-based features of binding poses.

The publication "Fenoterol and dobutamine as SARS-CoV-2 main protease inhibitors: A virtual screening study" by Bolelli Kayhan et al (JOURNAL OF MOLECULAR STRUCTURE, ELSEVIER AMSTERDAM, NL, vol. 1228, 13) explores fenoterol and dobutamine as potential SARS-CoV-2 main protease inhibitors through virtual screening methods. It highlights the docking scores and interactions of these compounds with the protease, suggesting their potential as COVID-19 treatments. The research discusses the methodology, results, and pharmacokinetic properties of the identified compounds.

**SUMMARY**

In view of these, some embodiments of the instant disclosure provide a drug ranking method and a drug ranking system. The present invention and the preferred embodiments are apparent from the appendant set of claims.

In some embodiments of the instant disclosure, a drug ranking method is provided and is executed by a processing unit. The drug ranking method is configured to rank a plurality of drugs according to a protein target. The drug ranking method comprises: according to a plurality of benchmark drugs from the plurality of drugs, a plurality of benchmark drug target proteins corresponding to the benchmark drugs, and a plurality of true binding poses and a plurality of decoy poses generated by docking the drugs with the benchmark drug target proteins, obtaining a statistics distribution pair for each of at least one feature, wherein the statistics distribution pair of each of the at least one feature comprises a true binding pose statistics distribution and a decoy pose statistics distribution; docking the protein target for each of the drugs to obtain a plurality of poses and at least one feature value for each of the poses, wherein the at least one feature value corresponds to the at least one feature; according to the at least one feature value of each of the poses and the statistics distribution pair of each of the at least one feature, obtaining a score of each of the poses for each of the drugs so as to obtain a plurality of pose scores of each of the drugs; and ranking the drugs according to the pose scores of each of the drugs, wherein the at least one feature comprises a distance between one of the poses and at least one active site residue, wherein the distance between one of the poses and the at least one active site residue is a distance between a center of mass of heavy atoms of one of the drugs and a center of mass of heavy atoms of all the active site residues.

In some embodiments of the instant disclosure, a drug ranking system is provided. The drug ranking system comprises a processing unit. The processing unit is configured to execute the aforementioned drug ranking method according to a protein target to rank a plurality of drugs.

Based on the above, in some embodiments of the instant disclosure, a drug ranking method and a drug ranking system, are provided. Hence, through the consideration of at least one feature of the pose, an accurate drug ranking method is provided. Furthermore, a comparison method for drug ranking method and a correction method for drug ranking method are also provided. In some embodiments of the instant disclosure, a method of selecting drugs that could be repurposed for treating diseases or symptoms (e.g., those caused by SARS-CoV-2 infection) other than their known indications.

BRIEF DESCRIPTION OF THE DRAWINGS

The disclosure will become more fully understood from the detailed description given herein below for illustration only, and thus not limitative of the disclosure, wherein:

Fig. 1 illustrates a block diagram of a drug ranking system according one embodiment of the instant disclosure;

Fig. 2 illustrates a schematic view showing the distance between the center of mass of the drug pose and the center of mass of the assigned target residue;

Fig. 3A illustrates a schematic graph showing the preliminary true binding pose statistics distribution and the preliminary decoy pose statistics distribution which correspond to the binding affinity according to one embodiment of the instant disclosure;

Fig. 3B illustrates a schematic view showing the preliminary true binding pose statistics distribution and the preliminary decoy pose statistics distribution which correspond to the distance between the pose and the assigned target residue according to one embodiment of the instant disclosure;

Fig. 3C illustrates a schematic view showing the preliminary true binding pose statistics distribution and the preliminary decoy pose statistics distribution which correspond to the size of the pose cluster according to one embodiment of the instant disclosure;

Fig. 3D illustrates a schematic view showing the preliminary true binding pose statistics distribution and the preliminary decoy pose statistics distribution which correspond to the distance between the pose and the active site residue according to one embodiment of the instant disclosure;

Fig. 4A illustrates a schematic graph showing the preliminary true binding pose statistics distribution and the preliminary decoy pose statistics distribution which correspond to the binding affinity according to one embodiment of the instant disclosure, wherein the binding affinity is calculated based on different approaches;

Fig. 4B illustrates a schematic view showing the preliminary true binding pose statistics distribution and the preliminary decoy pose statistics distribution which correspond to the distance between the pose and the assigned target residue according to one embodiment of the instant disclosure, wherein the distance between the pose and the assigned target residue is measured based on different approaches;

Fig. 4C illustrates a schematic view showing the preliminary true binding pose statistics distribution and the preliminary decoy pose statistics distribution which correspond to the size of the pose cluster according to one embodiment of the instant disclosure, wherein the size of the pose cluster is obtained based on different distance definitions;

Fig. 5 illustrates a flowchart of a drug ranking method according to one embodiment of the instant disclosure;

Fig. 6 illustrates a flowchart of a drug ranking method according to one embodiment of the instant disclosure;

Fig. 7 illustrates a flowchart of a drug ranking method according to one embodiment of the instant disclosure;

Fig. 8 illustrates a block diagram of a comparison system for drug ranking method;

Fig. 9 illustrates a flowchart of a comparison method for drug ranking method;

Fig. 10 illustrates a block diagram of a correction system for drug ranking method;

Fig. 11 illustrates a flowchart of a correction method for drug ranking method;

Fig. 12 illustrates a flowchart of the score calculation of the drug ranking method according to one embodiment of the instant disclosure;

Fig. 13 illustrates a staining results of the plaque assay for determining the anti-SARS-CoV-2 activity of the test drugs selected by the drug ranking method of the embodiments of the instant disclosure; and

Fig. 14 illustrates the anti-SARS-CoV-2 activity of the test drugs as calculated by quantification of the plaque assay results in Fig. 13.

## DETAILED DESCRIPTION

The foregoing and other technical contents, features, and effects of the instant disclosure can be clearly presented below in detailed description of the embodiments with reference to the drawings. Proportions or sizes of the components in the drawings expressed in an exaggerated, omitted, or in a general manner are used to help a person skilled in the art to understand and read, and are not intended to limit restraint conditions under which the instant disclosure can be implemented and therefore have no technical significance. Any modification to the structure, change to the proportional relationship or adjustment on the size should fall within the scope of the technical content disclosed by the instant disclosure without affecting the effects and the objectives that can be achieved by the present invention. The same reference numerals are used to indicate the same or similar components in all of the accompanying drawings. The term "connect" provided in the following embodiments may refer to any direct or indirect, or wired or wireless connection manners.

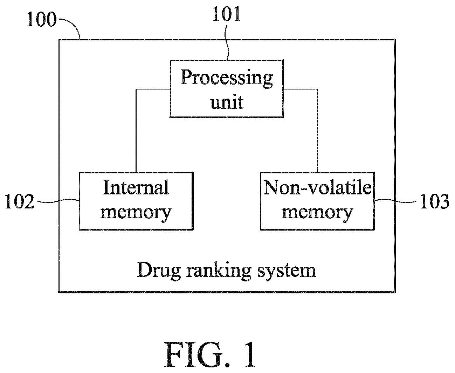

Fig. 1 illustrates a block diagram of a drug ranking system according one embodiment of the instant disclosure.

Please refer to Fig. 1. The drug ranking system 100 comprises a processing unit 101, an internal memory 102, and a non-volatile memory 103. The internal memory 102 may be for example a random-access memory (RAM), and the internal memory 102 is configured to temporarily store the intermediate results during the calculation. The processing unit 101 may comprise a single processor; the processing unit 101 may be configured as several processors to increase the computation speed.

In the following paragraphs, the drug ranking method and cooperative operations between the modules of the drug ranking system 100 are described along with the drawings.

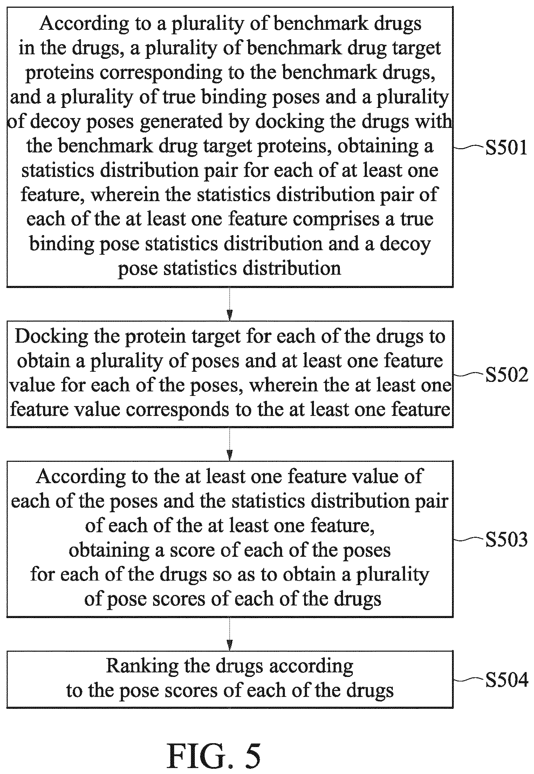

Fig. 5 illustrates a flowchart of a drug ranking method according to one embodiment of the instant disclosure. Please refer to Fig. 1 and Fig. 5 at the same time. The processing unit 101 is configured to execute the steps S501-S504 so as to rank a plurality of drugs according to a protein target.

In some embodiments of the instant disclosure, the drugs are for example 2016 drugs which are already approved by FDA. These 2016 FDA-approved drugs are recorded in the MedChemExpress (MCE) FDA-approved drug database (Cat. No.: HY-L022) and the Screen-Well® FDA-approved drug database (version 1.5) of the Enzo Life Science company. The protein target is SARS-CoV-2 nsp16 (PDB code: 6wks, chain A), and the assigned target residue is S-adenosyl methionine (SAM) (residue ID: 301).

In some embodiments, the drugs are the 2016 FDA-approved drugs. The benchmark drugs are recorded in the training set shown in Table 1 below.

Table 1

| PDB ID | Chain ID[2] | Seq. Length | Target Name | Related diseases | Drug name | Chemical ID | Binding Affinity | Active site residues or Functional site residues |
|---|---|---|---|---|---|---|---|---|
| Training set (number: 16) | | | | | | | | |
| 3g0b | A | 740 | Dipeptidyl peptidase 4 | Diabetes) | Alogliptin | T22 | IC50: 7 nM | Y547, S630, Y631, D708, H740 |
| 4mxo | A | 286 | Proto-oncogene tyrosine-protein kinase Src | Chronic myeloid leukemia (CML) | Bosutinib | DB8 | Kd: 0.73 nM | K295, E310, D386, D404, F405 |
| 4mkc | A | 367 | ALK tyrosine kinase receptor | Non-small cell lung cancers (NSCLC) | Ceritinib | 4MK | IC50: 0.2 nM | D1249, R1253, N1254 |
| 5p9i | A | 279 | Tyrosine-protein kinase BTK | Chronic lymphocytic leukemia (CLL) | Ibrutinib | 1E8 | - | D521, R525, N526, D539 |
| 3wzd | A | 309 | Vascular endothelial growth factor receptor 2 (VEGF 2) | Cancers | Lenvatinib | LEV | Kd: 2.1 nM | D1028, R1032, N1033, D1046 |
| 2rgu | A | 734 | Dipeptidyl peptidase 4 | Diabetes | Linagliptin | 356 | IC50: 0.1 nM | Y547, S630, Y631, D708, H740 |
| 2p16 | A | 234 | Activated blood coagulation factor X | Thrombotic diseases | Apixaban | GG2 | Ki: 0.08 nM | H57, D102, S195 |

| | | | | | | | | | D1028, R1032, N1033, D1046 |
|---|---|---|---|---|---|---|---|---|---|
| 4ag8 | A | 316 | Vascular endothelial growth factor receptor 2 (VEGF 2) | Cancers | Axitinib | AXI | Ki: 1.1 nM | | D1028, R1032, N1033, D1046 |
| 4xi3 | A | 243 | Estrogen receptor | Breast cancer | Bazedoxifene acetate | 29S | IC50: 0.6 nM | | D351, E353, R394 |
| 5csw | A | 282 | Serine/threonine-protein kinase B-raf | Cancers | Dabrafenib | P06 | IC50: 0.4 nM | | D576, K578, D594 |
| 4tvj | A | 368 | Poly [ADP-ribose] polymerase 2, (PARP 2) | Cancers | Olaparib | 09L | Kd: 0.28 nM | | S470, Y473, E558 |
| 2w26 | A | 234 | Activated blood coagulation factor X | Thrombotic diseases | Rivaroxaban | RIV | Ki: 0.4 nM | | H57, D102, S195 |
| 4rzv | A | 281 | Serine/threonine-protein kinase B-raf | Cancers | Vemurafenib | 032 | IC50: 4 nM | | D576, K578, D594 |
| 3lxk | A | 327 | Tyrosine-protein kinase JAK3 | Inflammatory diseases | Tofacitinib | MI1 | Ki: 0.2 nM | | K855, E871, D949 |
| 5ds3 | A | 271 | Poly [ADP-ribose] polymerase 1 (PARP 1) | Cancers | Olaparib | 09L | IC50: 0.9 nM | | S904, Y907, E988 |
| 2euf | B | 308 | Cyclin homolog | Cancers | Palbociclib | LQQ | IC50: 3.8 nM | | K43, E61, D145, D163 |
| Test set (number: 4) | | | | | | | | | |
| 2wgj | A | 306 | Hepatocyte growth factor receptor | Cancers | Crizotinib | VGH | Ki: 2 nM | | D1204, R1208, N1209, D1222 |
| 6nec | A | 314 | Proto-oncogene tyrosine-protein kinase receptor | Thyroid and non-small cell lung cancer | Nintedanib | XIN | - | | D874, R878, N879, D892 |
| 4zau | A | 330 | Epidermal growth factor receptor (EGFR) | Lung cancer | Osimertinib | YY3 | IC50: 1.6 nM | | D837, R841, N842, D855 |
| 4bjc | A | 240 | Poly [ADP-ribose] polymerase tankyrase-2 (PARP5b) | Cancers | Rucaparib | RPB | IC50: 14 nM | | S1068, Y1071, E1138 |

In Table 1, the target protein recited in the target protein column of the training set is the benchmark drug target protein corresponding to the benchmark drug. For example, the benchmark drug recited in Table 1 is Alogliptin, and the benchmark drug target protein corresponding to the benchmark drug Alogliptin is Dipeptidyl peptidase 4. Moreover, the drugs recited in the test set of Table 1 is referred to as the test set drug, and the target protein recited in the target protein column of Table 1 is the test set drug target protein corresponding to the set drug.

The benchmark drug and the benchmark drug target protein are referred as a drug-target complex. The 20 complex structures recited in Table 1 were extracted from the Protein Data Bank (PDB) (rcsb.org; related information can be found in Berman, H.M., Westbrook J., Feng Z., Gilliland G., Bhat T.N., Weissig H., Shindyalov I.N., Bourne P.E., 2000. The Protein Data Bank. Nucleic Acids Research 28, 235-242.). Firstly, 48 chemical IDs of FDA drugs that were newly approved during 2010-2016 were collected, where these drugs were gathered in the article published by Westbrook et al (Westbrook, J.D., Burley, S.K., 2019. How Structural Biologists and the Protein Data Bank Contributed to Recent FDA New Drug Approvals. Structure 27, 211-217.). For simplicity, the drugs reported to target the same target proteins were excluded. Among these 48 FDA-approved drugs, 44 drugs were included in the 2016 FDA-approved drug library. Then, the PDB IDs in which the structures were co-crystallized with one of the 44 drugs are fetched, and eventually 20 drug-target complexes recited in Table 1 can be achieved. The FDA-approved drug in the complex structure is referred to as "true binder".

It is noted that, although in the aforementioned embodiment, the 16 FDA-approved drugs recited in Table 1 are taken as the benchmark drugs, the instant disclosure is not limited thereto. In general, as long as the drug-target protein binding mode of the complex structure of a drug has been found, the drug can be taken as the benchmark drug. The drug-target protein binding mode of the complex structure of a drug may be obtained by (including but not limited to) experimental methods or computer prediction.

It is noted that, the column "Active site residues or Functional site residues" in Table 1 recites the residue ID of the active site residues or functional site residues of the target proteins of the drugs in Table 1. Wherein, the residues perform one of the functions listed below and the conservation score $\geqq 7$ which counts with Consurf DB are the active site residues (related information about Consurf DB can be found in Ben Chorin A., Masrati G., Kessel A., Narunsky A., Sprinzak J., Lahav S., Ashkenazy H. and Ben-Tal N. (2020). ConSurf-DB: An accessible repository for the evolutionary conservation patterns of the majority of PDB proteins. Protein Science 29:25-267): 1. direct involvement in the catalytic mechanism, e.g. as a nucleophile; 2. exerting an effect on another residue or water molecule which is directly involved in the catalytic mechanism which aids catalysis (e.g. by electrostatic or acid-base action); 3. stabilization of a proposed transition-state intermediate; and 4. exerting an effect on a substrate or cofactor which aids catalysis, e.g. by polarizing a bond which is to be broken, including steric and electrostatic effects (related information of the definitions of the active site residue can be found in Gail J. Bartlett, Craig T. Porter, Neera Borkakoti, Janet M. Thornton (2002), Analysis of Catalytic Residues in Enzyme Active Sites, Journal of Molecular Biology, Volume 324, Issue 1 , 105-121).

If a protein does not have an active site (e.g. substrate receptor), then the residues whose heavy atom is less than 4 Å far from the heavy atom of the drug on the x-ray-solved structure are chosen, and further the top three residues with the conservation score which counts with Consurf DB are picked as the functional site residues. If the picked residues have the same score, then the distance between the heavy atoms of the drug and the heavy atoms of residue are ordered decreasingly, and the three residues with the shortest distance are selected and defined as functional site residues.

The target protein of the complex structure with the PDB ID 4xi3 is a receptor and does not have an active site. Therefore, in the column "Active site residues or Functional site residues", the functional site residues for the target protein of the complex structure with the PDB ID 4xi3 are recited. In Table 1, for rest of the complex structures, the active site residues of the target proteins are recited in the columns "Active site residues or Functional site residues", respectively.

In this embodiment, in order to generate a pose and a feature value of a certain feature for the pose, the processing unit utilizes a docking software to dock the 16 complex structures recited in the training set of Table 1 with the 2016 FDA-approved drugs, and the sampling for several poses is allowed (owing to the performance limitation, in the embodiments of the instant disclosure, sampling 20 poses is allowed).

For the sake of convenience, the poses which are allowed to be sampled upon utilizing the docking software to dock a certain drug and a certain protein is referred to as the poses corresponding to the certain drug and the certain protein.

In some embodiments, the docking software may be AutoDock Vina. During the operation of AutoDock Vina, the docking box is set to cover the whole structure of the target protein with an additional 5 Å margin patched on each side of the docking box. The exhaustiveness was set to 50 to provide accurate screening results within a reasonable time. In some embodiments, the docking software may be Dock or Glide.

In this embodiment, the true binding pose of the true binder is defined as the pose of the true binder and the corresponding target protein (e.g., the true binder is Alogliptin, and the corresponding target protein is Dipeptidyl peptidase 4) that among all poses has the shortest distance between the center of mass of the complex structure and the center of mass of the heavy atoms of the true binder in the co-crystallized positions. Therefore, for each of the true binders, only one true binding pose exists. The poses which are obtained by docking the 16 complex structures of the training set with the 2016 FDA-approved drugs and are not the true binding pose are referred to as the decoys.

For example, in this embodiment, the number of the true binders is 16 (recited in the training set of Table 1). In the case that sampling 20 poses is allowed, when drug target proteins corresponding to the 16 true binders are respectively docked with the 2016 FDA-approved drugs, 16 true binding poses are obtained, and 20x16x2016-16 decoys are obtained.

In the step S501, according to a plurality of benchmark drugs in the drugs, a plurality of benchmark drug target proteins corresponding to the benchmark drugs, and a plurality of true binding poses and a plurality of decoy poses generated by docking the drugs with the benchmark drug target proteins, the processing unit 101 obtains a statistics distribution pair for each of at least one feature, wherein the statistics distribution pair of each of the at least one feature comprises a true binding pose statistics distribution and a decoy pose statistics distribution.

In some embodiments of the instant disclosure, the at least one feature comprises a binding affinity. The binding affinity may be defined as an original value (hereinafter, affinity) predicted by the docking software or may be defined as a value of an original value predicted by the docking software divided by a square root of the number of drug heavy atoms of a current calculating drug (hereinafter, normalized affinity). Therefore, in these embodiments, in the step S501, the processing unit 101 obtains the true binding pose statistics distribution and the decoy pose statistics distribution corresponding to the binding affinity.

In the step S502, the processing unit 101 docks the protein target for each of the drugs to obtain a plurality of poses and at least one feature value for each of the poses, wherein the at least one feature value corresponds to the at least one feature.

Taking an embodiment as an example (where in the embodiment, the drugs are 2016 FDA-approved drugs, the protein target is SARS-CoV-2 nsp16, and sampling 20 poses is allowed), the processing unit 101 docks the 2016 FDA-approved drugs with the protein target (in this embodiment, the protein target is SARS-CoV-2 nsp1). Therefore, for each of the 2016 FDA-approved drugs, 20 poses are generated. Each of the poses has a binding affinity value corresponding to the binding affinity (which also can be referred as the feature value corresponding to the binding affinity).

In the step S503, the processing unit 101 obtains a score of each of the poses for each of the drugs so as to obtain a plurality of poses scores of each of the drugs according to at least one feature value of each of the poses and the statistics distribution pair of each of the at least one features.

Taking the aforementioned embodiment (where in the embodiment, the drugs are 2016 FDA-approved drugs, the protein target is SARS-CoV-2 nsp 16, and sampling 20 poses is allowed) as an example, after the processing unit 101 docks the 2016 FDA-approved drugs with the protein target (in this embodiment, the protein target is SARS-CoV-2 nsp1), 20 poses are generated for each of the 2016 FDA-approved drugs. Each of the poses has a binding affinity value corresponding to the binding affinity (which also can be referred as the feature value corresponding to the binding affinity). Then, the processing unit 101 obtains 20 pose scores for each of the 2016 FDA-approved drugs according to the feature value of the pose corresponding to the binding affinity and the true binding pose statistics distribution and the decoy pose statistics distribution corresponding to the binding affinity obtained in the step S501.

Last, in the step S504, the processing unit 101 ranks the drugs according to the pose scores of each of the drugs. Taking the foregoing embodiment as an example, after the processing unit 101 obtains 20 pose scores for each of the 2016 FDA-approved drugs, the processing unit 101 then ranks the 2016 FDA-approved drugs according to the 20 pose scores of each of the 2016 FDA-approved drugs.

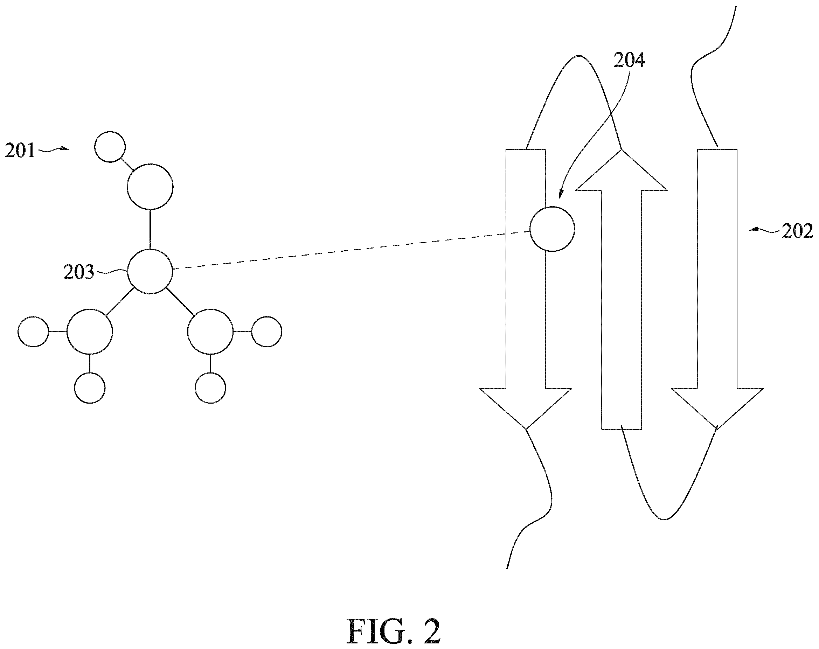

According to the invention, the at least one feature comprises a distance between one of the poses and at least one active site residue, wherein the distance between one of the poses and the at least one active site residue is a distance between a center of mass of heavy atoms of one of the drugs and a center of mass of heavy atoms of all the active site residues. Fig. 2 illustrates a schematic view showing the distance between the center of mass of the drug pose and the center of mass of the assigned target residue. Please refer to Fig. 2. In some embodiments, the distance between the pose and the assigned target residue is obtained by measuring the distance between the center of mass 203 of the drug pose 201 and the center of mass 204 of the protein target residue 202. It should be noted that, the distance between the pose and the assigned target residue is the shortest distance between any heavy atom of the drug pose and the center of mass of the assigned target residue.

As mentioned above, when a certain drug is docked with a certain protein by using the docking software, several poses are sampled, and these poses can be further clustered into clusters based on their distance from each other. Clustering the docking poses into groups is to approximate the entropy contribution by the size of the clusters, where a large cluster suggested a less entropy loss during the drug binding process. In some embodiments of the instant disclosure, the processing unit 101 adopts the hierarchical clustering as the clustering algorithm, where the distance in the hierarchical clustering is defined by the distance between the center of mass (COMS) of two poses (termed as "COM-based clustering") and the cutoff parameter of the hierarchical clustering is 4 Å. In some embodiments of the instant disclosure, the processing unit 101 adopts the "adjacency" of poses to cluster the poses (termed as "adjacency-based clustering", which will be described later). In adjacency-based clustering, poses were clustered based on whether any of their heavy atoms come within 1 Å. Then, the contact map of 20 poses x 20 poses was used to identify the connected components. The processing unit 101 can adopt the improved version of Tarjan's algorithm implemented in SciPy library of python to identify the connected components.

In this embodiment, the size of a pose cluster of a pose is defined as the number of the poses having high binding affinities, where the poses with top 10 binding affinities are defined as the poses having high binding affinities. Of course, the definition of the poses having high binding affinities may be changed to the posed with top 3, top 5, or top 15 binding affinities, the instant disclosure are not limited thereto.

In some embodiments of the instant disclosure, the at least one feature comprises the size of the pose cluster.

According to the invention, the feature comprises a distance between one of the poses and at least one active site residue. The distance between one of the poses and the at least one active site residue is a distance between the center of mass of the heavy atoms of one of the drugs and the center of mass of the heavy atoms of all the active site residues.

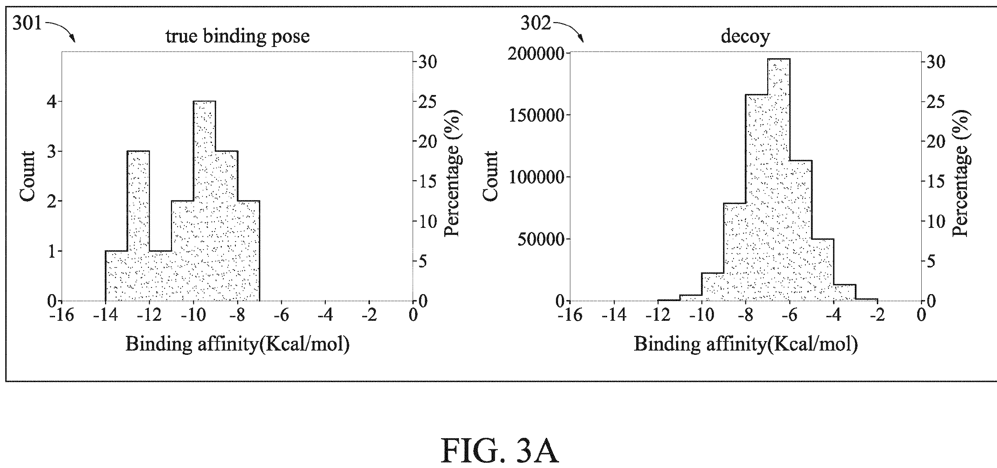

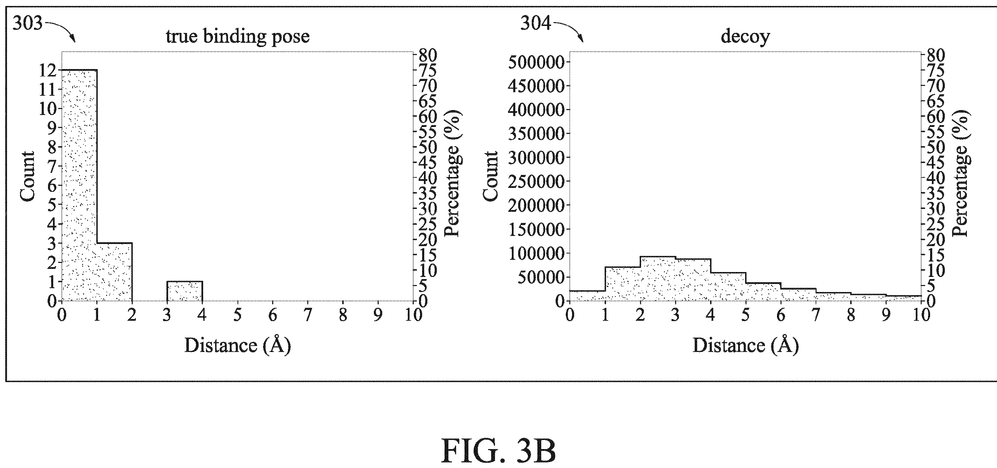

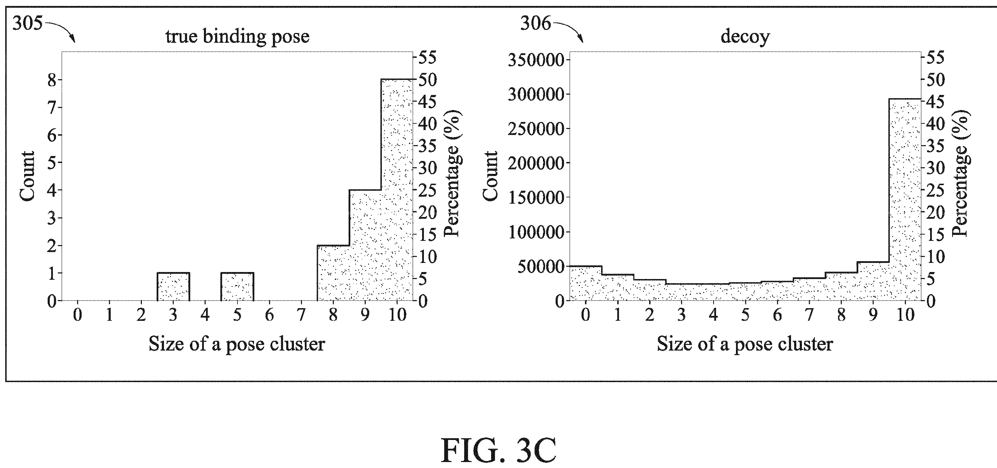

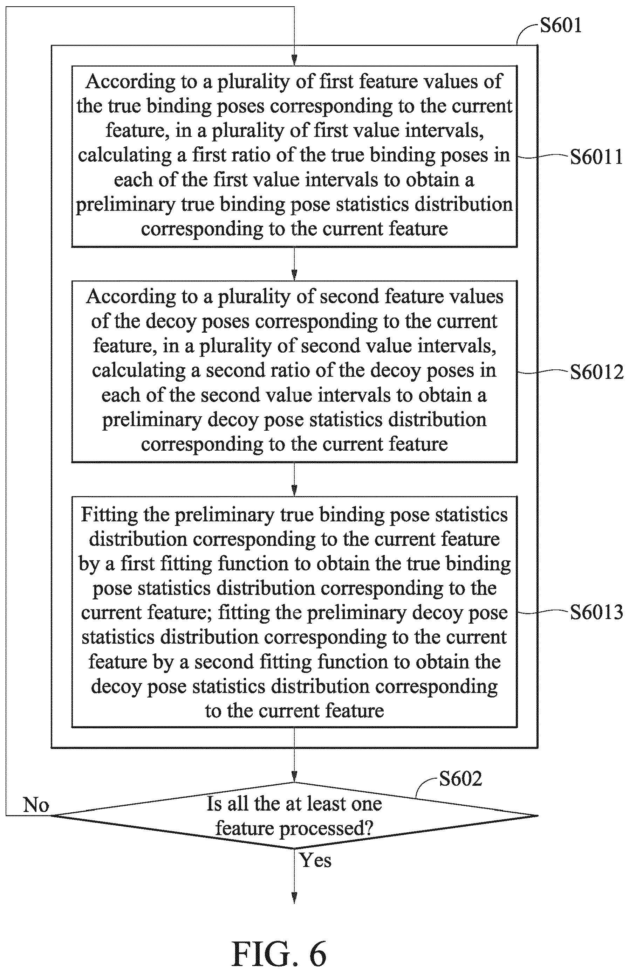

Fig. 3 A illustrates a schematic graph showing the preliminary true binding pose statistics distribution and the preliminary decoy pose statistics distribution which correspond to the binding affinity according to one embodiment of the instant disclosure. Fig. 3B illustrates a schematic view showing the preliminary true binding pose statistics distribution and the preliminary decoy pose statistics distribution which correspond to the distance between the pose and the assigned target residue according to one embodiment of the instant disclosure. Fig. 3C illustrates a schematic view showing the preliminary true binding pose statistics distribution and the preliminary decoy pose statistics distribution which correspond to the size of the pose cluster according to one embodiment of the instant disclosure. Fig. 6 illustrates a flowchart of a drug ranking method according to one embodiment of the instant disclosure. Please refer to Fig. 1, Fig. 3A to Fig. 3C, and Fig. 6 at the same time.

In the embodiment shown in Fig. 6, the step S501 comprises a step S601 and a step S602. The step S601 comprises a step S6011, a step S6012, and a step S6013. For describe the step S601 and the step S602, an embodiment is taken as an example (where in the embodiment, the drugs are 2016 FDA-approved drugs, the protein target is SARS-CoV-2 nsp16, the assigned target residue is S-adenosyl methionine (SAM) (residue ID: 301), the benchmark drugs are the 16 FDA-approved drugs which are recited in the training set of Table 1, sampling 20 poses is allowed, and the features are the binding affinity, the distance between the pose and the assigned target residue, and the size of the pose cluster). It is further noted that, in this example, 16 true binding poses are obtained, and 20x16x2016-16 decoys are obtained.

In the step S601, the processing unit 101 executes the step S6011, the step S6012, and the step S6013 for the current feature of the features. In the step S6011, according to a plurality of first feature values of the true binding poses corresponding to the current feature, in a plurality of first value intervals, the processing unit 101 calculates a first ratio of the true binding poses in each of the first value intervals to obtain a preliminary true binding pose statistics distribution corresponding to the current feature.

According to the foregoing illustrative example, if the current feature is the binding affinity (in this embodiment, the original value predicted by the docking software), then in the first value intervals ([-1,0), [-2,-1), [-3,-2)..., [-16,-15), and so on), the processing unit 101 calculates the ratio of the first feature value corresponding to the binding affinity of the true binding poses of the benchmark drugs and the benchmark drug target protein. Taking the preliminary true binding pose statistics distribution 301 in Fig. 3A for illustration, the values of two binding affinities of the true binding poses are within the interval [-8,-7), so that the processing unit 101 counts the number of the interval [-8,-7) as two, and the values of three binding affinities of the true binding poses are with the interval [-9,-8), so that the processing unit 101 counts the number of the interval [-9,-8) as three, and vise versa. Eventually, a statistics distribution like the preliminary true binding pose statistics distribution 301 illustrated in Fig. 3A can be obtained. Then, the processing unit 101 calculates the first ratio for each of the first value intervals according to the number corresponding to each of the first value intervals so as to obtain the ratio as indicated at the right side of the preliminary true binding pose statistics distribution 301 illustrated in Fig. 3A. Hence, the preliminary true binding pose statistics distribution corresponding to the binding affinity, like the preliminary true binding pose statistics distribution 301 illustrated in Fig. 3A, can be obtained.

In the step S6012, according to a plurality of second feature values of the true binding poses corresponding to the current feature, in a plurality of second value intervals, the processing unit 101 calculates a second ratio of the decoy poses in each of the second value intervals to obtain a preliminary decoy pose statistics distribution corresponding to the current feature.

According to the foregoing illustrative example, the current feature is the binding affinity (in this embodiment, the original value predicted by the docking software), then in the second value intervals ([-1,0), [-2,-1), [-3,-2)..., [-16,-15), and so on), the processing unit 101 calculates the ratio of the second feature value corresponding to the binding affinity of the decoy poses. Taking the preliminary decoy pose statistics distribution 302 in Fig. 3A for illustration, no binding affinities of the decoy poses are within the interval [-1,0), therefore the processing unit 101 counts the number of the interval [-1,-0) as zero, and vice versa. Eventually, a statistics distribution like the preliminary decoy pose statistics distribution 302 illustrated in Fig. 3A can be obtained. Then, the processing unit 101 calculates the second ratio for each of the second value intervals according to the number corresponding to each of the second value intervals so as to obtain the ratio as indicated at the right side of the preliminary decoy pose statistics distribution 302 illustrated in Fig. 3A. Hence, the preliminary decoy pose statistics distribution corresponding to the binding affinity, like the preliminary decoy pose statistics distribution 302 illustrated in Fig. 3A, can be obtained.

In the step S6013, the processing unit 101 fits the preliminary true binding pose statistics distribution corresponding to the current feature by a first fitting function to obtain the true binding pose statistics distribution corresponding to the current feature, wherein the fitting values of the ratios are the probability values corresponding to the feature values of the true binding pose statistics distribution corresponding to the current feature. Furthermore, the processing unit 101 fits the preliminary decoy pose statistics distribution corresponding to the current feature by a second fitting function to obtain the decoy pose statistics distribution corresponding to the current feature, wherein the fitting values of the ratios are the probability values corresponding to the feature values of the decoy pose statistics distribution corresponding to the current feature.

According to the foregoing illustrative example, after the processing unit 101 obtains the preliminary true binding pose statistics distribution and the preliminary decoy pose statistics distribution corresponding to the binding affinity (as the preliminary true binding pose statistics distribution 301 and the preliminary decoy pose statistics distribution 302 illustrated in Fig. 3A), the processing unit 101 fits the preliminary true binding pose statistics distribution 301 corresponding to the binding affinity by the first fitting function and fits the preliminary decoy pose statistics distribution 302 corresponding to the binding affinity by the second fitting function.

In some embodiments of the instant disclosure, the processing unit 101 fits the preliminary true binding pose statistics distribution 301 corresponding to the binding affinity by a skew-normal distribution function and fits the preliminary decoy pose statistics distribution 302 corresponding to the binding affinity by a normal distribution function.

In the step S602, the processing unit 101 determines whether all the features are processed. If yes, the processing unit 101 executes the step S502; if not, the processing unit 101 executes the step S6011, the step S6012, and the step S6013 of the step S601.

According to the foregoing illustrative example, the features comprise the binding affinity, the distance between the pose and the assigned target residue, and the size of the pose cluster. Therefore, the processing unit 101 repeatedly executes the step S6011, the step S6012, and the step S6013 until all the features are processed, so that the processing unit 101 obtains the preliminary true binding pose statistics distribution 303 and the preliminary decoy pose statistics distribution 304 which correspond to the distance between the pose and the assigned target residue

(in this embodiment, the distance between the center of mass of the drug pose and the center of mass of the protein target residue is taken as the distance between the pose and the assigned target residue), and the processing unit 101 also obtains the preliminary true binding pose statistics distribution 305 and the preliminary decoy pose statistics distribution 306 which correspond to the size of the pose cluster (in this embodiment, the size of the pose cluster is determined based on the adjacency-based clustering).

In some embodiments, the processing unit 101 fits the preliminary true binding pose statistics distribution 303 corresponding to the distance between the pose and the assigned target residue by a half-gaussian function and fits the preliminary decoy pose statistics distribution 304 corresponding to the distance between the pose and the assigned target residue by the half-gaussian function.

In some embodiments, the processing unit 101 fits the preliminary true binding pose statistics distribution 305 corresponding to the size of the pose cluster by a half-gaussian function and fits the preliminary decoy pose statistics distribution 306 corresponding to the size of the pose cluster by the half-gaussian function.

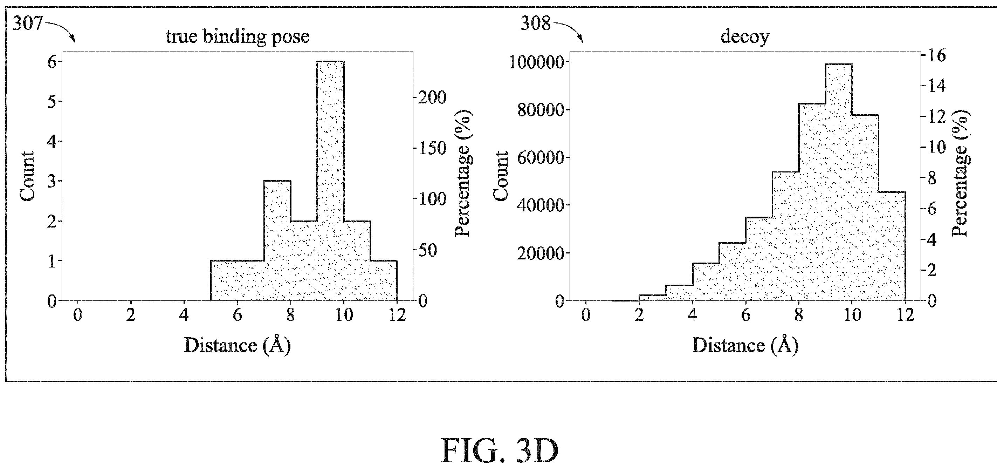

Fig. 3D illustrates a schematic view showing the preliminary true binding pose statistics distribution and the preliminary decoy pose statistics distribution which correspond to the distance between the pose and the active site residue according to one embodiment of the instant disclosure. The data of the preliminary true binding pose statistics distribution 307 and the preliminary decoy pose statistics distribution 308 are presented as Table 3D-1 and Table 3D-2 below:

Table 3D-1

| Distance (Å) | Count | Percentage (%) |
|---|---|---|
| [5,6) | 1 | 6.25% |
| [6,7) | 1 | 6.25% |
| [7,8) | 3 | 18.75% |
| [8,9) | 2 | 12.50% |
| [9,10) | 6 | 37.50% |
| [10,11) | 2 | 12.50% |
| [11,12) | 1 | 6.25% |

Table 3D-2

| Distance (Å) | Count | Percentage (%) |
|---|---|---|
| [1,2) | 76 | 0.01% |
| [2,3) | 2312 | 0.36% |
| [3,4) | 6488 | 1.01% |
| [4,5) | 15511 | 2.41% |
| [5,6) | 24588 | 3.82% |
| [6,7) | 35169 | 5.47% |
| [7,8) | 54339 | 8.45% |
| [8,9) | 82905 | 12.89% |
| [9,10) | 99497 | 15.47% |
| [10,11) | 78015 | 12.13% |
| [11,12) | 45871 | 7.13% |

In some embodiments of the instant disclosure, the processing unit 101 fits the preliminary true binding pose statistics distribution 307 corresponding to the distance between the pose and the active site residue by a skew-normal distribution function and fits the preliminary decoy pose statistics distribution 308 corresponding to the distance between the pose and the active site residue by the skew-normal distribution function.

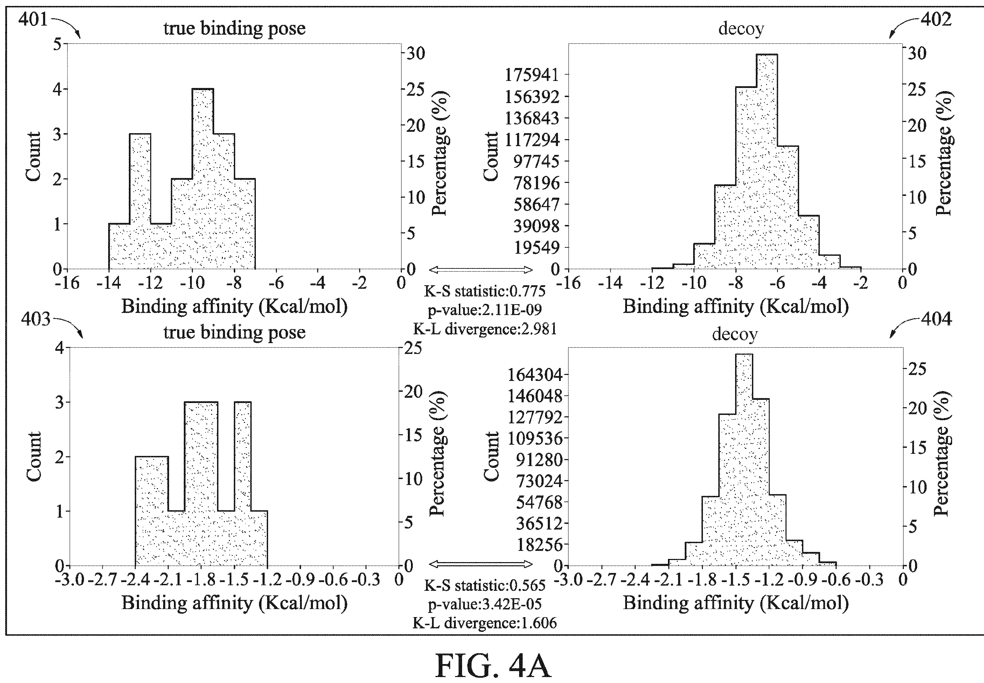

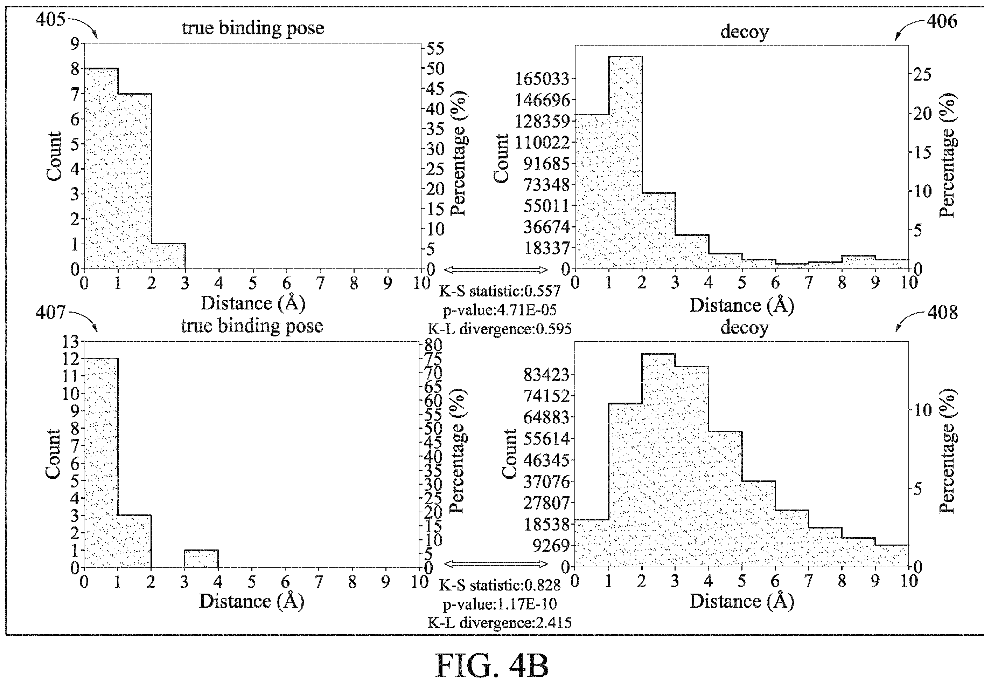

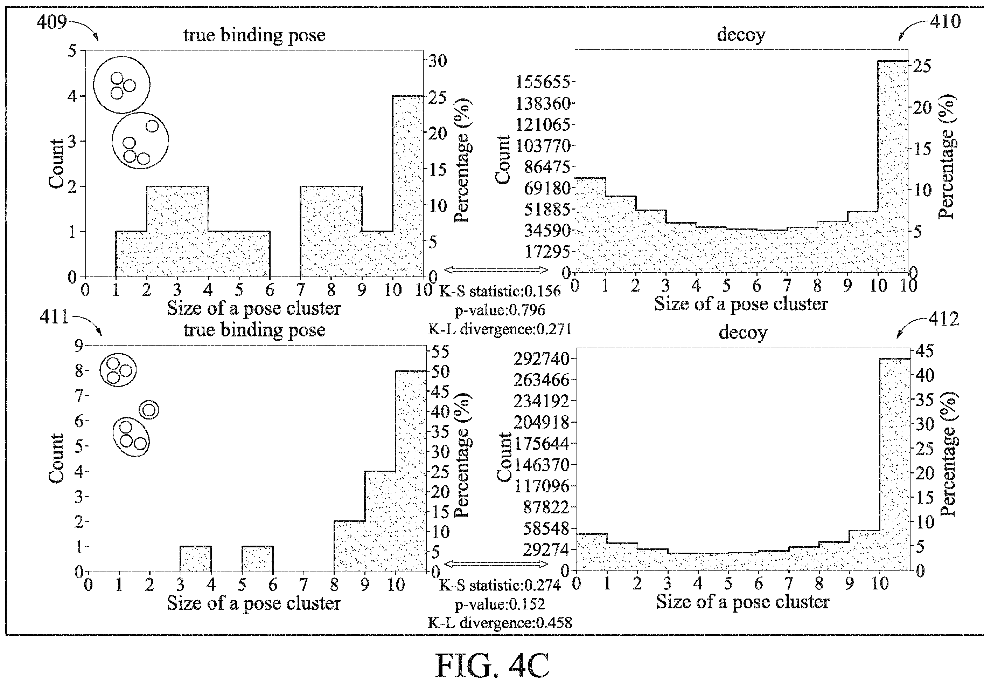

Fig. 4A illustrates a schematic graph showing the preliminary true binding pose statistics distribution and the preliminary decoy pose statistics distribution which correspond to the binding affinity according to one embodiment of the instant disclosure, wherein the binding affinity is calculated based on different approaches. Fig. 4B illustrates a schematic view showing the preliminary true binding pose statistics distribution and the preliminary decoy pose statistics distribution which correspond to the distance between the pose and the assigned target residue according to one embodiment of the instant disclosure, wherein the distance between the pose and the assigned target residue is measured based on different approaches. Fig. 4C illustrates a schematic view showing the preliminary true binding pose statistics distribution and the preliminary decoy pose statistics distribution which correspond to the size of the pose cluster according to one embodiment of the instant disclosure, wherein the size of the pose cluster is obtained based on different distance definitions. Please refer to Fig. 4A, Fig. 4B, and Fig. 4C at the same time.

As mentioned above, upon calculating the binding affinity of the pose, the original value predicted by the docking software (that is, the affinity) may be adopted, or a value of the original value predicted by the docking software divided by a square root of the number of the drug heavy atoms of a current calculating drug (that is, the normalized affinity) can be adopted. In some embodiments of the instant disclosure, for the certain drugs (such as the 2016 FDA-approved drugs), the benchmark drugs in the certain drugs, and the benchmark drug target protein corresponding to the benchmark drugs, the processing unit 101 firstly adopts the original value predicted by the docking software (that is, the affinity) as the binding affinity of the pose and obtains a first group of the preliminary true binding pose statistics distribution corresponding to the binding affinity (as the preliminary true binding pose statistics distribution 401 illustrated in Fig. 4A) and the preliminary decoy pose statistics distribution corresponding to the binding affinity (as the preliminary decoy pose statistics distribution corresponding 402 illustrated in Fig. 4A) according to the steps S601-S602.

Next, the processing unit 101 adopts the value of the original value predicted by the docking software divided by a square root of the number of the drug heavy atoms of a current calculating drug (that is, the normalized affinity) as the binding affinity of the pose and obtains a second group of the preliminary true binding pose statistics distribution corresponding to the binding affinity (as the preliminary true binding pose statistics distribution 403 illustrated in Fig. 4A) and the preliminary decoy pose statistics distribution corresponding to the binding affinity (as the preliminary decoy pose statistics distribution corresponding 404 illustrated in Fig. 4A) according to the steps S601-S602.

Then, the processing unit 101 calculates at least one statistical property of the first group of the preliminary true binding pose statistics distribution corresponding to the binding affinity (as the preliminary true binding pose statistics distribution 401 illustrated in Fig. 4A) and the preliminary decoy pose statistics distribution corresponding to the binding affinity (as the preliminary decoy pose statistics distribution corresponding 402 illustrated in Fig. 4A) and the at least one statistical property of the second group of the preliminary true binding pose statistics distribution corresponding to the binding affinity (as the preliminary true binding pose statistics distribution 403 illustrated in Fig. 4A) and the preliminary decoy pose statistics distribution corresponding to the binding affinity (as the preliminary decoy pose statistics distribution corresponding 404 illustrated in Fig. 4A). Then, the processing unit 101 determines whether to adopt the affinity or the normalized affinity as the binding affinity of the pose according to the at least one statistical property.

The at least one statistical property comprises the Kolmogorov-Smirov test (K-S test), the p-value in the distribution of the K-S statistic, and the Kullback-Leibler (K-L) divergence. A larger K-S test value or a larger K-L divergence value indicates that the difference between the preliminary true binding pose statistics distribution and the preliminary decoy pose statistics distribution is larger; a smaller p-value in the distribution of the K-S statistic indicates that the difference between the preliminary true binding pose statistics distribution and the preliminary decoy pose statistics distribution is larger. The processing unit chooses one of the affinity and the normalized affinity as the binding affinity of the pose based on that the chosen value allows a larger difference between the preliminary true binding pose statistics distribution and the preliminary decoy pose statistics distribution.

In some embodiments of the instant disclosure, the processing unit 101 takes one of the K-S test, the p-value in the distribution of the K-S statistic, and the K-L divergence alone as the basis for demining whether to adopt the affinity or the normalized affinity as the binding affinity of the pose. In some embodiments of the instant disclosure, the processing unit 101 comprehensively considers the K-S test, the p-value in the distribution of the K-S statistic, and the K-L divergence (for example, the processing unit 101 chooses one of the K-S test, the p-value in the distribution of the K-S statistic, and the K-L divergence which allows a larger difference between the preliminary true binding pose statistics distribution and the preliminary decoy pose statistics distribution) as the basis for demining whether to adopt the affinity or the normalized affinity as the binding affinity of the pose.

In the embodiment shown in Fig. 4A, in the first group, the K-S test value is 0.775, the p-value in the distribution of the K-S statistic is 2.11E-9, and the K-L divergence is 2.981; in the second group, the K-S test value is 0.565, the p-value in the distribution of the K-S statistic is 3.42E-5, and the K-L divergence is 1.606. Therefore, in this embodiment, no matter the basis is one or all of the K-S test, the p-value in the distribution of the K-S statistic, and the K-L divergence, the processing unit 101 adopts the affinity as the binding affinity of the pose.

Please refer to Fig. 4B and Fig. 4C at the same time. The preliminary true binding pose statistics distribution 405 and the preliminary decoy pose statistics distribution 406 are a statistics distribution pair corresponding to the distance between the pose and the assigned target residue (where the shortest distance between any heavy atom of the drug pose and the center of mass of the assigned target residue is taken the distance between the pose and the assigned target residue). The preliminary true binding pose statistics distribution 407 and the preliminary decoy pose statistics distribution 408 are a statistics distribution pair corresponding to the distance between the pose and the assigned target residue (where the distance between the center of mass of the drug pose and the center of mass of the protein target residue is taken as the distance between the pose and the assigned target residue).

The preliminary true binding pose statistics distribution 409 and the preliminary decoy pose statistics distribution 410 are a statistics distribution pair corresponding to the size of the pose cluster (where the size of the pose cluster is determined based on the COM-based clustering). The preliminary true binding pose statistics distribution 411 and the preliminary decoy pose statistics distribution 412 are a statistics distribution pair corresponding to the size of the pose cluster (where the size of the pose cluster is determined based on the adjacency-based clustering).

Based on the same reason as mentioned above, the processing unit 101 adopts the distance between the center of mass of the drug pose and the center of mass of the protein target residue as the distance between the pose and the assigned target residue, and the processing unit 101 adopts the size of the pose cluster based on the adjacency-based clustering.

It should be noted that, the application of the statistical property is not limited to determine the features of the binding affinity, the distance between the pose and the assigned target residue, and the size of the pose cluster; the statistical property can also be applied to determine other features with several definitions based on the aforementioned manners. In other words, the above method can be applied to two and more than two definitions of a feature to select the better definition of the feature.

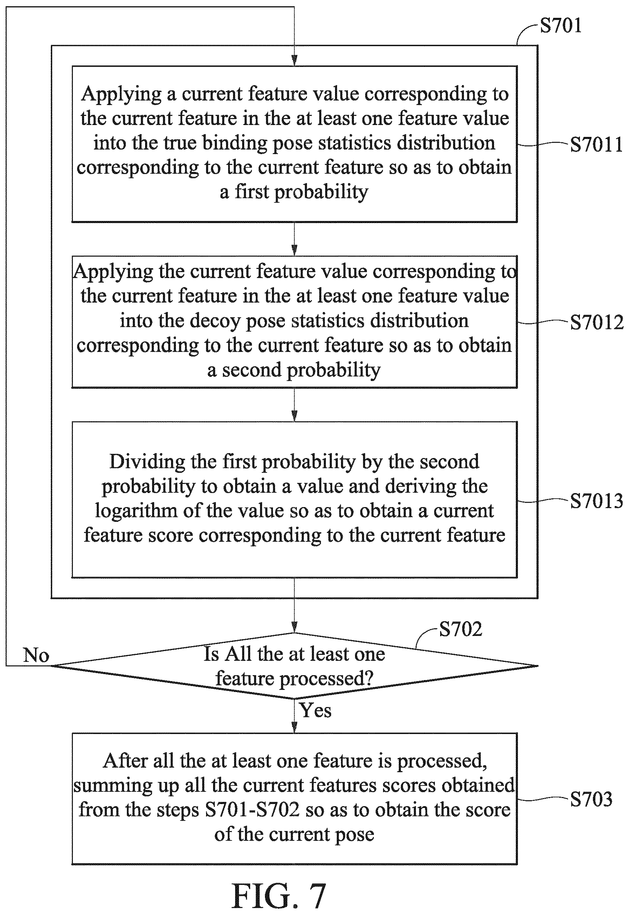

Fig. 7 illustrates a flowchart of a drug ranking method according to one embodiment of the instant disclosure. Please refer to Fig. 5 and Fig. 7 at the same time. In the embodiment shown in Fig. 7, the step S503 comprises a step S701, a step S702, and a step S703. An embodiment is taken as an example (where in the embodiment, the drugs are 2016 FDA-approved drugs, the protein target is SARS-CoV-2 nsp16, the benchmark drugs are the 16 FDA-approved drugs which are recited in the training set of Table 1, sampling 20 poses is allowed, and the features are the binding affinity, the distance between the pose and the assigned target residue, and the size of the pose cluster). In this example, 16 true binding poses are obtained, and 20x16x2016-16 decoys are obtained. Moreover, the processing unit 101 already obtains three group of statistics distribution pairs corresponding to the binding affinity, the distance between the pose and the assigned target residue, and the size of the pose cluster.

As mentioned above, in the step S502, the processing unit 101 already docks the protein target for each of the drugs to obtain a plurality of poses and at least one feature value for each of the poses, wherein the at least one feature value corresponds to the at least one feature. For example, the processing unit docks Alogliptin among the 2016 FDA-approved drugs with the protein target SARS-CoV-2 nsp16 to obtain 20 poses, and each of the poses has a feature value corresponding to the binding affinity, a feature value corresponding to the distance between the pose and the assigned target residue, and a feature value corresponding to the size of the pose cluster.

For each of the drugs, the processing unit 101 executes the step S701, the step S702, and the step S703 for a current pose of the poses. In the step S701, a step S7011, a step S7012, and a step S7013 are executed. In the step S7011, the processing unit 101 puts a current feature value corresponding to the current feature in the at least one feature value into the true binding pose statistics distribution corresponding to the current feature so as to obtain a first probability. In the step S7012, the processing unit 101 puts a current feature value corresponding to the current feature in the at least one feature value into the decoy pose statistics distribution corresponding to the current feature so as to obtain a second probability. In the step S703, the processing unit 101 divides the first probability by the second probability to obtain a value and deriving the logarithm of the value so as to obtain a current feature score corresponding to the current feature.

According to the foregoing illustrative example, the current pose has a feature value corresponding to the binding affinity, a feature value corresponding to the distance between the pose and the assigned target residue, and a feature value corresponding to the size of the pose cluster. In the case that the current feature is the binding affinity, then in the step S7011, the feature value corresponding to the binding affinity is putted into the true binding pose statistics distribution corresponding to the binding affinity to obtain the first probability, for example, 0.125. Next, in the step S7012, the feature value corresponding to the binding affinity is putted into the decoy pose statistics distribution corresponding to the binding affinity to obtain the second probability, for example, 0.28. in the step S7013, the first probability is divided by the second probability to obtain a value, and the logarithm of the value is derived so as to obtain a current feature score corresponding to the binding affinity, that is $log_{10} \frac{0.125}{0.28} = -0.35$.

In the step S702, the processing unit 101 determines whether all the features are processed. If yes, the processing unit 101 executes the step S703; if not, the processing unit 101 executes the step S701 until all the features are processed. According to the foregoing illustrative example, the processing unit 101 repeatedly executes the step S701 until the feature of the binding affinity, the feature of the distance between the pose and the assigned target residue, and the feature of the size of the pose cluster are processed.

In the step S703, after all features are processed by the processing unit 101, the processing unit 101 sums up all the current feature scores obtained from the steps S701-S702 so as to obtain the score of the current pose. According to the foregoing illustrative example, the processing unit 101 eventually obtains a current feature score corresponding to the binding affinity (e.g., -0.35), a current feature score corresponding to the distance between the pose and the assigned target residue (e.g., -0.1), and a current feature score corresponding to the size of the pose cluster (e.g., -0.2). Then, the processing unit 101 calculates the pose score of the current pose to be (-0.35)+(-0.1)+(-0.2)=-0.65.

If x denotes the pose, f denotes the feature, $Q_f(x)$ denotes the feature value, $P_f^T$ denotes the first probability obtained by applying the feature value corresponding to the binding affinity to the true binding pose statistics distribution corresponding to the binding affinity, and $P_f^F$ denotes the second probability obtained by applying the feature value corresponding to the binding affinity to the decoy pose statistics distribution corresponding to the binding affinity, then the score of the current pose can be denoted as:

$$\sum_f log \frac{P_f^T(Q_f(x))}{P_f^F(Q_f(x))} \ldots \ldots \ldots (1).$$

In some embodiments, the step S504 comprises ranking the drugs in a high-to-low manner according to a highest score among the pose scores of each of the drugs. According to the foregoing illustrative example, for each of the drugs, 20 poses are obtained, and each of the poses has a pose score. Therefore, for each of the drugs, 20 pose scores are obtained. For each of the drugs, the processing unit 101 chooses the highest pose score among the 20 pose scores as the highest score, and then the processing unit 101 ranks the drugs according to the highest score for each of the drugs.

The method that the steps S501-S504 including the steps S701-S703 are referred to as the log-odds score (LOD score) method.

In some embodiments of the instant disclosure, the processing unit 101 ranks a plurality of drugs by the following Ad hoc method for drug ranking based on the aforementioned three features of the binding affinity, the distance between the pose and the assigned target residue, and the size of the pose cluster.

The Ad hoc method for drug ranking is composed of two stages. In the first stage, a representative pose was chosen among a plurality of poses (e.g., the 20 poses of the preceding embodiment) for each drug by the following steps. First, the poses (e.g., the 20 poses of the preceding embodiment) are divided into two groups based on the feature value of the distance between the pose and the assigned target residue of each pose, the first group having a feature value within 5 Å and the second group having a feature value greater than 5 Å. The first group precedes the second group. As mentioned before, the aforementioned poses (e.g., the 20 poses of the preceding embodiment) can be divided into clusters according to the hierarchical clustering, and one of these clusters is the largest cluster. Based on the aforementioned largest cluster, each of these two groups can be further divided into two classes, the first of which belongs to the aforementioned largest cluster and the second of which does not. The first class precedes the second class. Thus, the aforementioned poses (e.g., the 20 poses of the preceding embodiment) can be sequentially divided into 4 classes: a first class of the first cluster, a second class of the first group, a first class of the second group, and a second class of the second group. In each of the four classes formed, the poses are then ranked according to the binding affinity. The first ranked pose in the first class of the first group is selected as the representative pose for the drug.

In the second stage, the drugs are divided into two groups based on the feature value of the distance between the pose and the assigned target residue of the representative pose selected in the first stage for each drug, with the first group having a feature value within 5 Å and the second group having a feature value greater than 5 Å. The first group precedes the second group. All drugs in the two groups were then ranked according to their binding affinity to obtain the rank of the plurality of drugs.

It is worth noting that the Ad hoc method for drug ranking may rank drugs based on the aforementioned two features of the binding affinity and the distance between the pose and the assigned target residue. The difference with the aforementioned the Ad hoc method for drug ranking based on the aforementioned binding affinity, distance between the pose and the assigned target residue, and size of the pose cluster is that, in the first stage, the aforementioned poses (e.g., the 20 poses of the preceding embodiment) are divided into two groups based on the feature value of the distance between the pose and the assigned target residue of each pose, the first group having a feature value within 5 Å and the second group having a feature value greater than 5 Å. The first group precedes the second group. The first group and the second group are no longer divided into two classes. The ranking of the poses is then done directly according to the binding affinity. The first ranked pose in the first group is selected as the representative pose for the drug. After that, the second stage described above is proceed.

It is worth noting that the Ad hoc method for drug ranking may rank drugs based on only the aforementioned one feature of the binding affinity. The difference with the aforementioned the Ad hoc method for drug ranking based on the aforementioned binding affinity, distance between the pose and the assigned target residue, and size of the pose cluster is that, in the first stage, the ranking of the poses is then done directly according to the binding affinity. Then the first ranked pose is selected as the representative pose for the drug. After that, the second stage described above is proceed. In the second stage, all drugs is then ranked according to the binding affinity of the representative pose of each drugs selected in the first stage to obtain the rank of the plurality of drugs.

In some embodiments of the instant disclosure, the processing unit 101 ranks a plurality of drugs by the following feature ranking score (FRS) method for drug ranking based on the aforementioned three features of the binding affinity, the distance between the pose and the assigned target residue, and the size of the pose cluster.

The first step of the feature ranking score (FRS) method is to calculate an FRS for each of the poses generated by docking each one of the drugs and protein target, where

$$\text{FRS} = \sum_{f=1}^{3}\left[1 - \frac{(R_f - 1)}{N}\right] \dots \dots \dots (2),$$

f represents the feature (including the binding affinity, the distance between the pose and the assigned target residue, and the size of the pose cluster), $R_f$ represents the ranking of the feature value of feature f of the pose in all the poses corresponding to all drugs, where a lower drug affinity and drugs' distances to target sites, or a larger size of pose clusters score correspond to a higher rank, starting from one. N is the total number of the poses from all the plurality of drugs.

The second step of the feature ranking score (FRS) method is to use the pose with the highest FRS in each drug as the representative pose for that drug and ranks all the drugs according to the FRS of the representative pose for each drug.

It is worth noting that the aforementioned feature ranking score method ranks all drugs based on the aforementioned three features of the binding affinity, the distance between the pose and the assigned target residue, and the size of the pose cluster, the aforementioned feature ranking score method can also rank all drugs based on a subset of the three features of the binding affinity, the distance between the pose and the assigned target residue, and the size of the pose cluster, as long as the number of sums taken is modified. The aforementioned feature ranking score method can also be applied to other different features.

In some embodiments of the instant disclosure, the processing unit 101 ranks a plurality of drugs by the following weighted feature ranking score (weighted FRS) method for drug ranking based on the aforementioned three features of the binding affinity, the distance between the pose and the assigned target residue, and the size of the pose cluster. The weighted feature ranking score replaces the FRS of Equation (2) with the following FRS score (Equation (3)):

$$\text{FRS} = \sum_{f=1}^{3} w_f \left[1 - \frac{(R_f - 1)}{N}\right] \dots \dots \dots (3),$$

where $w_f$ is the feature weight corresponding to feature f and $0 < w_f < 1$ and $\Sigma_f w_f = 1$.

Like the aforementioned feature ranking scoring method, the aforementioned weighted feature ranking score method can also rank all drugs based on a subset of the three features of the binding affinity, the distance between the pose and the assigned target residue, and the size of the pose cluster, as long as the number of sums taken is modified. The aforementioned weighted feature ranking score method can also be applied to other different features.

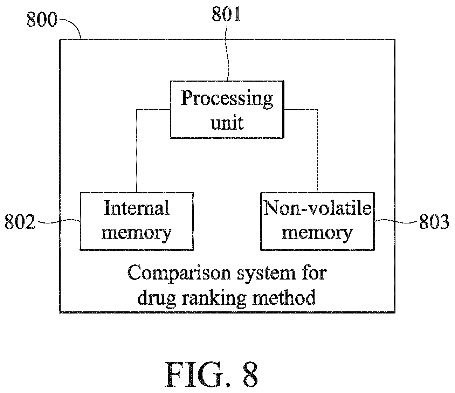

Fig. 8 illustrates a block diagram of a comparison system for drug ranking method. Please refer to Fig. 8. The comparison system for drug ranking method 800 comprises a processing unit 801, an internal memory 802, and a non-volatile memory 803. The internal memory 802 may be for example a random-access memory (RAM), and the internal memory 802 is configured to temporarily store the intermediate results during the calculation. The processing unit 801 may comprise a single processor; the processing unit 801 may be configured as several processors to increase the computation speed.

In the following paragraphs, the comparison method for drug ranking method and cooperative operations between the modules of the comparison system for drug ranking method 800 are described along with the drawings.

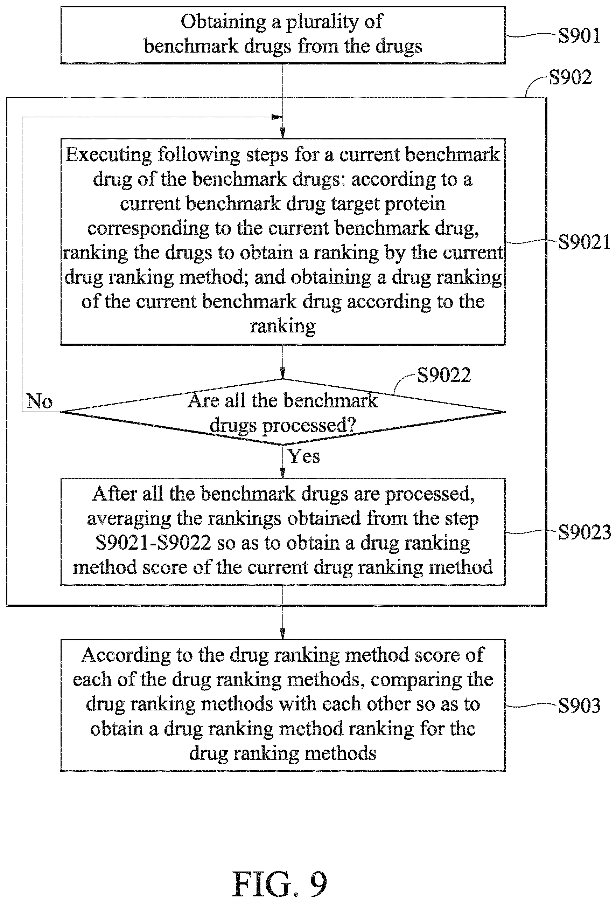

Fig. 9 illustrates a flowchart of a comparison method for drug ranking method. Please refer to Fig. 8 and Fig. 9 at the same time. The processing unit 801 is configured to execute the steps S901-S903 so as to compare a plurality of drug ranking methods, wherein the drug ranking methods are configured to rank a plurality of drugs according to a protein target. The step S902 comprises a step S9021, a step S9022, and a step S9023. In the step S901, the processing unit 801 obtains a plurality of benchmark drugs from the drugs. In the step S902, the processing unit 801 executes the step S9021, the step S0922, and the step S9023 for the current drug ranking method of the drug ranking methods. In the step S9021, according to a current benchmark drug target protein corresponding to a current benchmark drug as the protein target, the processing unit 801 ranks the drugs to obtain a ranking for each of the drugs by the current drug ranking method. Then, the processing unit 801 obtains a drug ranking of the current benchmark drug according to the ranking for each of the drugs. In other words, in this embodiment, the processing unit 801 ranks the drugs according to the current benchmark drug target protein by the current drug ranking method to obtain the ranking of the current benchmark drug (if the current drug ranking method performs well, theoretically, the ranking of the current benchmark drug has a smaller number (higher rank)).

In the step S9022, the processing unit 801 determines whether all the benchmark drugs are processed. If not, then the processing unit 801 repeatedly executes the step S9021 until all the benchmark drugs are processed. If yes, the processing unit 801 executes the step S9023. In the step S9023, the processing unit 801 averages the rankings obtained from the steps S9021-S9022 so as to obtain a drug ranking method score of the current drug ranking method. If the current benchmark drug target protein order is 1, 2, 3, ... , and M, the drug ranking method score can be presented by following equation:

$$\mathrm{Rank}_{\mathrm{avg}} = \frac{1}{M} \sum_{j=1}^{M} r_j^T \qquad\qquad \ldots\ldots(4)$$

Wherein $\mathrm{Rank}_{\mathrm{avg}}$ denotes the drug ranking method score, M is a positive integer, and denotes the number of the benchmark drugs. $r_j^T$ denotes the drug ranking of a $T^{th}$ benchmark drug for a $j^{th}$ current benchmark drug target protein.

In the step S903, according to the drug ranking method score $\mathrm{Rank}_{\mathrm{avg}}$ of each of the drug ranking methods, the processing unit 801 compares the drug ranking methods with each other so as to obtain a drug ranking method ranking for the drug ranking methods.

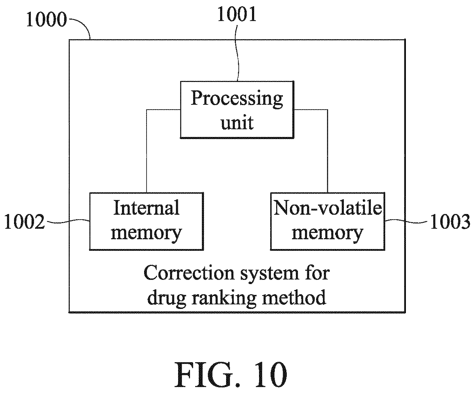

Fig. 10 illustrates a block diagram of a correction system for drug ranking method. Please refer to Fig. 10. The correction system for drug ranking method 1000 comprises a processing unit 1001, an internal memory 1002, and a non-volatile memory 1003. The internal memory 1002 may be for example a random-access memory (RAM), and the internal memory 1002 is configured to temporarily store the intermediate results during the calculation. The processing unit 1001 may comprise a single processor; the processing unit 1001 may be configured as several processors to increase the computation speed.

In the following paragraphs, the correction method for drug ranking method and cooperative operations between the modules of the correction system for drug ranking method 1000 are described along with the drawings.

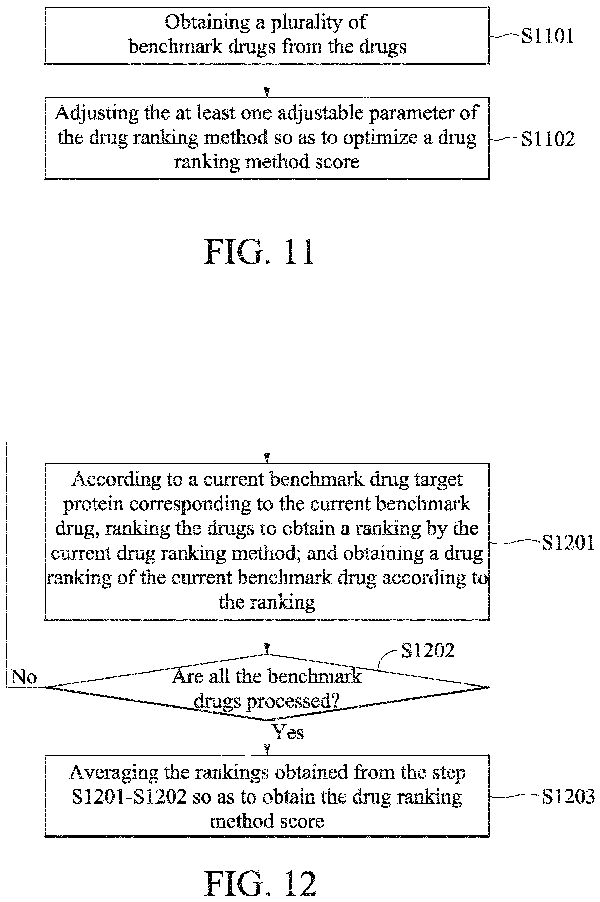

Fig. 11 illustrates a flowchart of a correction method for drug ranking method. Fig. 12 illustrates a flowchart of the score calculation of the drug ranking method. Please refer to Fig. 10, Fig. 11, and Fig. 12 at the same time. The processing unit 1001 is configured to execute the steps S1101-S1102 so as to correct a drug ranking method. The drug ranking method has at least one adjustable parameter, and the drug ranking method is configured to rank a plurality of drugs according to a protein target. In the step S1101, the processing unit 1001 obtains a plurality of benchmark drugs from the drugs. In the step S1102, the processing unit 1101 adjusts the at least one adjustable parameter of the drug ranking method so as to optimize a drug ranking method score of the drug ranking method. Wherein, the drug ranking method score is defined by the equation (4). The drug ranking method score can be obtained from the flowchart illustrated in Fig. 12. It is understood that, because the step S1201, the step S1202, and the step S1203 shown in Fig. 12 are respectively identical to the step S9021, the step S9022, and the step S9023, and thus details for the step S1201, the step S1202, and the step S1203 are not explained repeatedly again.

It should be noted that, owing to the computation limitation, to minimize the drug ranking method score, the processing unit 1001 cannot always provide all the possible values of the at least one adjustable parameter for all drug ranking methods. The term "optimize" indicates that the processing unit 1001 figures out at least one parameter value of the at least one adjustable parameter of the drug ranking method among finite possible values of the at least one adjustable parameter of the drug ranking method to make the drug ranking method score minimized among the finite possible values of the at least one adjustable parameter.

The at least one adjustable parameter of the drug ranking method correspond to different method models under the same ranking method concept. For example, the number of nodes which could be adjusted in a neural network, the number of layers in a neural network, the power of a adjustable term in a polynomial regression model, different distribution functions that fit the same statistical distribution, different kinds or numbers of features used in the same operation method (e.g., the aforementioned ad hoc method for drug ranking), etc.

In some embodiments of the instant disclosure, the aforementioned drug ranking method is the aforementioned weighted feature ranking scoring method, and the at least one adjustable parameter of the drug ranking method is the at least one feature weight of the weighted feature ranking scoring method. Using the aforementioned weighted feature ranking score (weighted FRS) method for drug ranking based on the aforementioned three features of the binding affinity, the distance between the pose and the assigned target residue, and the size of the pose cluster as an example, in some embodiments of the instant disclosure, the processing unit 101 applies grid search method to search for better values of

$\{w_f\}$ to optimize the drug ranking method score (Equation (4)) of the aforementioned drug ranking method. The step to search for better values of $\{w_f\}$ by grid search is to make any two feature weights $w_1$ and $w_2$ in $\{w_f\}$ as any values in the set $\{0, 0.1, 0.2, ..., 0.9, 1\}$ and search for better values in the set $(w_1, w_2, 1-w_1-w_2)$ exhaustively to optimize the drug ranking method score (Equation (4)) of the aforementioned drug ranking method.

In some embodiments of the instant disclosure, the processing unit 101 applies sampling search method to search for better values of $\{w_f\}$ to optimize the drug ranking method score (Equation (4)) of the aforementioned drug ranking method. The step to search for better values of $\{w_f\}$ by sampling search method is to sample $(w_1, w_2, w_3)$ according to the following equation (Equation (5)),

$$(w_1, w_2, w_3) = (0,0,1) + a(1,0,-1) + b(0,1,-1) \ldots \ldots (5),$$

where $a$ and b were sampled uniformly from 0 to 1, and keep the set $(w_1, w_2, w_3)$ where $w_3 >= 0$. 10,000 sets of such $(w_1, w_2, w_3)$ are sampled and the processing unit 101 search for better values in the sets exhaustively to optimize the drug ranking method score (Equation (4)) of the aforementioned drug ranking method.

Table 2 below shows the rank of the 16 benchmark drugs, the drug ranking method scores (Equation (4)) of the various drug ranking methods, and the success rates of the various drug ranking methods after ranking the 2016 FDA-approved drugs based on the benchmark drug target proteins of each of the 16 benchmark drugs using the various drug ranking methods disclosed in the preceding embodiments. The success rate is defined as the percentage of drugs in the 16 benchmark drugs that is ranked within the indicated top rank 100 (~5%) or 200 (~10%).

Table 2

| PDB ID | Drug name | Affinity | Affinity + Distance | Affinity +Distance +Clustering | LOD score | FRS rank (Affinity +Distance) | FRS rank (Affinity +Distance +Clustering) | Weighted FRS rank (Grid) | Weighted FRS rank (Sampling) |
|---|---|---|---|---|---|---|---|---|---|
| 3g0b | Alogliptin | 1030 | 1050 | 1060 | 770 | 1232 | 1390 | 1170 | 1181 |
| 4mxo | Bosutinib | 731 | 688 | 668 | 465 | 472 | 446 | 631 | 572 |
| 4mkc | Ceritinib | 78 | 61 | 54 | 5 | 2 | 154 | 5 | 5 |
| 5p9i | Ibrutinib | 113 | 89 | 89 | 76 | 183 | 244 | 135 | 167 |
| 3wzd | Lenvatinib | 22 | 16 | 12 | 4 | 24 | 16 | 9 | 10 |
| 2rgu | Linagliptin | 232 | 187 | 175 | 341 | 203 | 207 | 169 | 175 |
| 2p16 | Apixaban | 45 | 45 | 47 | 3 | 15 | 800 | 9 | 13 |
| 4ag8 | Axitinib | 1 | 1 | 1 | 1 | 3 | 2 | 2 | 2 |
| 4xi3 | Bazedoxifene | 2 | 2 | 2 | 2 | 6 | 482 | 3 | 3 |
| 5csw | Dabrafenib | 2 | 2 | 2 | 2 | 1 | 1 | 1 | 1 |
| 4tvj | Olaparib | 8 | 8 | 8 | 1 | 2 | 2 | 1 | 1 |
| 2w26 | Rivaroxaban | 86 | 81 | 91 | 6 | 13 | 594 | 29 | 24 |
| 4rzv | Vemurafenib | 1 | 1 | 1 | 1 | 1 | 1 | 1 | 1 |
| 3lxk | Tofacitinib | 223 | 186 | 205 | 327 | 107 | 856 | 71 | 73 |
| 5ds3 | Olaparib | 4 | 4 | 4 | 3 | 1 | 1 | 1 | 1 |

| 2euf | Palbociclib | 53 | 48 | 42 | 12 | 36 | 34 | 14 | 23 |
|---|---|---|---|---|---|---|---|---|---|
| Drug ranking method scores (Equation (4)) | | 164.44 | 154.31 | 153.81 | 126.19 | 143.81 | 326.88 | 140.69 | 140.75 |
| Success rate (< 100, ~ 5%)[7] | | 68.8% (11) | 75.0% (12) | 75.0% (12) | 75.0% (12) | 68.8% (11) | 43.8% (7) | 75.0% (12) | 75.0% (12) |
| Success rate (< 200, ~ 10%)[7] | | 75.0% (12) | 87.5% (14) | 81.3% (13) | 75.0% (12) | 81.3% (13) | 50.0% (8) | 87.5% (14) | 87.5% (14) |

The **"Affinity"** column contains the results of applying the Ad hoc method for ranking 2016 FDA-approved drugs based on the binding affinity (the original value predicted by the docking software). The **"Affinity +** Distance " column contains the results of applying the Ad hoc method for ranking 2016 FDA-approved drugs based on the binding affinity (the original value predicted by the docking software) and the distance between the pose and the assigned target residue (the distance between the center of mass of the drug pose and the center of mass of the protein target residue is taken as the distance between the pose and the assigned target residue). The **"Affinity +Distance +Clustering"** column contains the results of applying the Ad hoc method for ranking 2016 FDA-approved drugs based on the binding affinity (the original value predicted by the docking software), the distance between the pose and the assigned target residue (the distance between the center of mass of the drug pose and the center of mass of the protein target residue is taken as the distance between the pose and the assigned target residue) and the size of the pose cluster (the size of the pose cluster is determined based on the adjacency-based clustering). The **"LOD score"** column contains the results of applying the log-odds score method for ranking 2016 FDA-approved drugs based on the binding affinity (the original value predicted by the docking software), the distance between the pose and the assigned target residue (the distance between the center of mass of the drug pose and the center of mass of the protein target residue is taken as the distance between the pose and the assigned target residue) and the size of the pose cluster (the size of the pose cluster is determined based on the adjacency-based clustering). The **"FRS rank (Affinity + Distance)"** column contains the results of applying the FRS method for ranking 2016 FDA-approved drugs based on the two features of binding affinity (the original value predicted by the docking software) and the distance between the pose and the assigned target residue (the distance between the center of mass of the drug pose and the center of mass of the protein target residue is taken as the distance between the pose and the assigned target residue). The **"FRS rank (Affinity +Distance +Clustering)"** column contains the results of applying the FRS method for ranking 2016 FDA-approved drugs based on the three features of the binding affinity (the original value predicted by the docking software), the distance between the pose and the assigned target residue (the distance between the center of mass of the drug pose and the center of mass of the protein target residue is taken as the distance between the pose and the assigned target residue) and the size of the pose cluster (the size of the pose cluster is determined based on the adjacency-based clustering). The **"Weighted FRS rank (Grid)"** column contains the results of applying the corrected weighted FRS method (with the feature weights 0.8, 0.2 and 0) for ranking 2016 FDA-approved drugs based on the three features of the binding affinity (the original value predicted by the docking software), the distance between the pose and the assigned target residue (the distance between the center of mass of the drug pose and the center of mass of the protein target residue is taken as the distance between the pose and the assigned target residue) and the size of the pose cluster (the size of the pose cluster is determined based on the adjacency-based clustering), wherein the feature weights of the corrected weighted FRS method are obtained by applying the aforementioned correction method for drug ranking method and the grid search method. The **"Weighted FRS rank (Sampling)"** column contains the results of applying the corrected weighted FRS method (with the feature weights 0.72470771, 0.27438212 and 0.00091017) for ranking 2016 FDA-approved drugs based on the three features of the binding affinity (the original value predicted by the docking software), the distance between the pose and the assigned target residue (the distance between the center of mass of the drug pose and the center of mass of the protein target residue is taken as the distance between the pose and the assigned target residue) and the size of the pose cluster (the size of the pose cluster is determined based on the adjacency-based clustering), wherein the feature weights of the corrected weighted FRS method are obtained by applying the aforementioned correction method for drug ranking method and the sampling search method.

It is also noteworthy that Table 3 below shows the rank of the 4 test set drugs, the drug ranking method scores (Equation (4)) of the various drug ranking methods, and the success rates of the various drug ranking methods after ranking the 2016 FDA-approved drugs based on the test set drug target proteins of each of the 4 test set drugs using the various drug ranking methods disclosed in the preceding embodiments. The success rate is defined as the percentage of drugs in the 4 test set drugs that is ranked within the indicated top rank 100 (~5%) or 200 (~10%). The success rate is the proportion of drugs in the top 100 (~5%) or 200 (~10%) of the four test sets that were ranked.

Table 3

| PDB ID | Drug name | Affinity | Affinity + Distance | Affinity +Distance +Clustering | LOD score[4] | FRS rank (Affinity +Distance) | FRS rank (Affinity +Distance +Clustering) | Weighted FRS rank (Grid) | Weighted FRS rank (Sampling) |
|---|---|---|---|---|---|---|---|---|---|
| 2wgj | Crizotinib | 41 | 38 | 39 | 2 | 1 | 339 | 1 | 1 |
| 6nec | Nintedanib | 21 | 20 | 17 | 3 | 1 | 1 | 1 | 1 |
| 4zau | Osimertinib | 585 | 738 | 712 | 98 | 470 | 301 | 739 | 650 |
| 4bjc | Rucaparib | 284 | 249 | 244 | 24 | 6 | 6 | 21 | 16 |
| Drug ranking method scores (Equation (4)) | | 232.75 | 261.25 | 253.0 | 31.75 | 119.5 | 161.75 | 190.5 | 167.0 |
| Success rate (< 100, ~ 5%)[7] | | 50.0% (2) | 50.0% (2) | 50.0% (2) | 100.0% (4) | 75.0% (3) | 50.0% (2) | 75.0% (3) | 75.0% (3) |
| Success rate (< 200, ~ 10%)[7] | | 50.0% (2) | 50.0% (2) | 50.0% (2) | 100.0% (4) | 75.0% (3) | 50.0% (2) | 75.0% (3) | 75.0% (3) |

Explicit solvent MD simulations using classical force fields optimized for biological molecules are recognized to better describe the protein-ligand binding dynamics in a timescale allowed for weak binders to dissociate (related information can be found in Huang, D., Caflisch, A., 2011. Small Molecule Binding to Proteins: Affinity and Binding/Unbinding Dynamics from Atomistic Simulations. ChemMedChem 6, 1578-1580; Liu, K., Kokubo, H., 2017. Exploring the Stability of Ligand Binding Modes to Proteins by Molecular Dynamics Simulations: A Cross-docking Study. Journal of Chemical Information and Modeling 57, 2514-2522 ; 以及 Mollica, L., Decherchi, S., Zia, S.R., Gaspari, R., Cavalli, A., Rocchia, W., 2015. Kinetics of protein-ligand unbinding via smoothed potential molecular dynamics simulations). The resulting trajectories further allow accurate binding free energy evaluation by MM/GBSA, where the molecular mechanics of protein-ligand complexes and a continuum solvent model accounting for the solvation energy calculation are considered suitable to assess relative binding affinity for a set of drugs against the same pocket (related information can be found in Ahinko, M., Niinivehmas, S., Jokinen, E., Pentikäinen, O.T., 2018. Suitability of MMGBSA for the selection of correct ligand binding modes from docking results. Chemical Biology & Drug Design 93, 522-538; Liu, P.-F., Tsai, K.-L., Hsu, C.-J., Tsai, W.-L., Cheng, J.-S., Chang, H.-W., Shiau, C.-W., Goan, Y.-G., Tseng, H.-H., Wu, C.-H., Reed, J.C., Yang, L.-W., Shu, C.-W., 2018. Drug Repurposing Screening Identifies Tioconazole as an ATG4 Inhibitor that Suppresses Autophagy and Sensitizes Cancer Cells to Chemotherapy. Theranostics 8, 830-845; Sun, H., Li, Y., Tian, S., Xu, L., Hou, T., 2014. Assessing the performance of MM/PBSA and MM/GBSA methods. 4. Accuracies of MM/PBSA and MM/GBSA methodologies evaluated by various simulation protocols using PDBbind data set. Phys. Chem. Chem. Phys. 16, 16719-16729; Yang, T., Wu, J.C., Yan, C., Wang, Y., Luo, R., Gonzales, M.B., Dalby, K.N., Ren, P., 2011. Virtual screening using molecular simulations. Proteins: Structure, Function, and Bioinformatics 79, 1940-1951; Zhang, X., Perez-Sanchez, H., C. Lightstone, F., 2017. A Comprehensive Docking and MM/GBSA Rescoring Study of Ligand Recognition upon Binding Antithrombin. Current Topics in Medicinal Chemistry 17, 1631-1639).

The MD simulations for user-selected poses were performed by the processing unit 101 using the OpenMM package (related information can be found in Eastman, P., Swails, J., Chodera, J.D., Mcgibbon, R.T., Zhao, Y., Beauchamp, K.A., Wang, L.-P., Simmonett, A.C., Harrigan, M.P., Stern, C.D., Wiewiora, R.P., Brooks, B.R., Pande, V.S., 2017. OpenMM 7: Rapid development of high performance algorithms for molecular dynamics. PLOS Computational Biology 13) in an explicit solvent. The simulation model of the protein target-drug binding pose complex was prepared using the LEaP program in AmberTools (related information can be found in Case, D.A., Belfon, K., Ben-Shalom, I.Y, Brozell, S.R., Cerutti, D.S., Cheatham, T.E., III, Cruzeiro, VW.D., Darden, T.A., Duke, R.E., Giambasu, G., Gilson, M.K., Gohlke, H. Goetz, A.W., Harris, R., Izadi, S., Kasavajhala, K., Kovalenko, A., Krasny, R., Kurtzman, T., Lee, T.S., LeGrand, S., Li, P., Lin, C., Liu, J., Luchko, T., Luo, R., Man, V., Merz, K.M., Miao, Y., Mikhailovskii, O., Monard, G., Nguyen, H., Onufriev, A., Pan, F., Pantano, S., Qi, R., Roe, D.R., Roitberg, A., Sagui, C., Schott-Verdugo, S., Shen, J., Simmerling, C.L., Skrynnikov, N., Smith, J., Swails, J., Walker, R.C., Wang, J., Wilson, L., Wolf, R.M., Wu, X., York, D.M. and Kollman, P.A., 2020. AMBER 2020, University of California, San Francisco). The complex was solvated using an explicit solvent of the TIP3P water model, with at least 10 Å of water layer patched on each side of the water box between the protein target and the box boundary. Sodium and chloride ions were used to neutralize the system to achieve a salt concentration of 100 mM. The system was first energy minimized for all the hydrogen, waters, and ions positions, leaving the remaining atoms restrained using a force constant of 10 kcal/mol/$Å^2$. This was followed by the energy minimization for atoms including also protein heavy atoms, leaving the protein C-alpha atoms and drug heavy atoms restrained using a force constant of 2 kcal/mol/Å$^2$. The system was then heated to 320 K in NVT ensemble for 1 ns and equilibrated for another 1 ns in NPT ensemble 310 K and 1 atm. The protein C-alpha atoms and drug heavy atoms were weakly restrained (0.1 kcal/mol/Å$^2$) during the NVT heating and NPT equilibration. The equilibrated system was then subject to a 10 ns simulation using the same condition as in the equilibration except for the removal of all restraints, and the snapshots were sampled for every 0.1 ns, resulting in a trajectory composed of 100 frames. Langevin dynamics with a collision frequency of 2 ps$^{-1}$ and a Monte Carlo Barostat with the volume rescaled every 25 time steps were applied for temperature and pressure controls, respectively. The particle mesh Ewald (PME) method was used to calculate the energy of non-bonded interaction with 10 Å as the distance cutoff. All the bonds involving hydrogens were constrained by SHAKE algorithm (related information can be found in Miyamoto, S., Kollman, P.A., 1992. Settle: An analytical version of the SHAKE and RATTLE algorithm for rigid water models. Journal of Computational Chemistry 13, 952-962). For the pre-relaxation of submitted protein structure or modeled structure from AlphaFold2, a similar protocol was used except no ligand was bound and the final NPT production run lasted for 1 ns. The binding free energy approximated by the enthalpy contribution between the target protein and drugs for each sampled snapshot was calculated by MM/GBSA methods (related information can be found in Genheden, S., Ryde, U., 2015. The MM/PBSA and MM/GBSA methods to estimate ligand-binding affinities. Expert Opinion on Drug Discovery 10, 449-461; Greenidge, P.A., Kramer, C., Mozziconacci, J.-C., Wolf, R.M., 2012. MM/GBSA Binding Energy Prediction on the PDBbind Data Set: Successes, Failures, and Directions for Further Improvement. Journal of Chemical Information and Modeling 53, 201-209; Kollman, P.A., Massova, I., Reyes, C., Kuhn, B., Huo, S., Chong, L., Lee, M., Lee, T., Duan, Y., Wang, W., Donini, O., Cieplak, P., Srinivasan, J., Case, D.A., Cheatham, T.E., 2000. Calculating Structures and Free Energies of Complex Molecules: Combining Molecular Mechanics and Continuum Models. Accounts of Chemical Research 33, 889-897; Massova, I., Kollman, P.A., 2000. Combined molecular mechanical and continuum solvent approach (MM-PBSA/GBSA) to predict ligand binding. Perspectives in Drug Discovery and Design 18, 113-135) with MMPBSA.py module (related information can be found in Miller, B.R., Mcgee, T.D., Swails, J.M., Homeyer, N., Gohlke, H., Roitberg, A.E., 2012. MMPBSA.py: An Efficient Program for End-State Free Energy Calculations. Journal of Chemical Theory and Computation 8, 3314-3321) in the AmberTools (related information can be found in Case, D.A., Belfon, K., Ben-Shalom, I.Y, Brozell, S.R., Cerutti, D.S., Cheatham, T.E., III, Cruzeiro, V.W.D., Darden, T.A., Duke, R.E., Giambasu, G., Gilson, M.K., Gohlke, H. Goetz, A.W., Harris, R., Izadi, S., Kasavajhala, K., Kovalenko, A., Krasny, R., Kurtzman, T., Lee, T.S., LeGrand, S., Li, P., Lin, C., Liu, J., Luchko, T., Luo, R., Man, V., Merz, K.M., Miao, Y., Mikhailovskii, O., Monard, G., Nguyen, H., Onufriev, A., Pan, F., Pantano, S., Qi, R., Roe, D.R., Roitberg, A., Sagui, C., Schott-Verdugo, S., Shen, J., Simmerling, C.L., Skrynnikov, N., Smith, J., Swails, J., Walker, R.C., Wang, J., Wilson, L., Wolf, R.M., Wu, X., York, D.M. and Kollman, P.A., 2020. AMBER 2020, University of California, San Francisco). The simulation results were summarized using the designed indicators, including the mean binding free energy from MM/GBSA calculation over sampled snapshots, the drug leaving time when the drug center of mass moving away from the target site more than 10 Å, and the largest distance of the drug COM to the target sites sampled during the simulations. A simulated drug got a higher rank if having a favorable mean binding free energy and staying long enough in the target site (or never leaving) during the 10 ns simulation. All the processing and analysis of the docking poses and MD trajectories were performed by the processing unit 101 using ParmEd (related information can be found in http://parmed.github.io/ParmEd), Cpptraj (related information can be found in Roe, D.R., Cheatham, T.E., 2013. PTRAJ and CPPTRAJ: Software for Processing and Analysis of Molecular Dynamics Trajectory Data. Journal of Chemical Theory and Computation 9, 3084-3095), PyTraj (related information can be found in Nguyen, H., Roe, D.R., Swails, J., Case, D.A., 2015. pytraj (https://github.com/Amber-MD/pytraj)), and MDAnalysis (related information can be found in Gowers, R., Linke, M., Barnoud, J., Reddy, T., Melo, M., Seyler, S., Domański, J., Dotson, D., Buchoux, S., Kenney, I., Beckstein, O., 2016. MDAnalysis: A Python Package for the Rapid Analysis of Molecular Dynamics Simulations. Proceedings of the 15th Python in Science Conference; and Michaud-Agrawal, N., Denning, E.J., Woolf, T.B., Beckstein, O., 2011. MDAnalysis: A toolkit for the analysis of molecular dynamics simulations. Journal of Computational Chemistry 32, 2319-2327).

In some embodiments of the instant disclosure, the drugs are for example 2016 drugs which are already approved by FDA, the protein target is SARS-CoV-2 nsp16, the assigned target residue is S-adenosyl methionine (SAM) (residue ID: 301), the benchmark drugs are the 16 FDA-approved drugs which are recited in the training set of Table 1, and sampling 20 poses is allowed. The processing unit 101 docks the 2016 FDA-approved drugs with the protein target (in this embodiment, the protein target is SARS-CoV-2 nsp1), respectively. The processing unit 101 apply the log-odds score method for ranking 2016 FDA-approved drugs based on the binding affinity (the original value predicted by the docking software), the distance between the pose and the assigned target residue (the distance between the center of mass of the drug pose and the center of mass of the protein target residue is taken as the distance between the pose and the assigned target residue) and the size of the pose cluster (the size of the pose cluster is determined based on the adjacency-based clustering).

Table 4 below shows the top-ranked 20 drugs based on the LOD scores derived from the docking results and their corresponding indicators inferred from 10 ns MD simulations, including the mean binding free energy, the drug leaving time, and the largest distance of the drug to the target sites during the simulations. The natural ligand, S-adenosyl methionine (SAM), serving as a methyl donor for the mRNA substrate and belonging to one of the FDA-approved drugs included in our drug library, was ranked 17 at the docking stage, which demonstrated the robustness and accuracy of our drug ranking method based on the LOD score. Confirmed by the 10 ns simulation, SAM was further prioritized to rank 2 with the mean binding free energy of -31.0 kcal/mol and the longest distance of 1.43 Å to the target site, suggesting the high affinity and stable binding of the natural ligand with nsp16.

Table 4

| Drug name | Rank | Highest pose score among the 20 pose scores | Affinity (kcal/mol) | Affinity rank | MD rank | Mean binding free energy (kcal/mol) | Drug leaving time (ns) | Longest distance to the target site (Å) |
|---|---|---|---|---|---|---|---|---|
| Enasidenib | 1 | 4.65 | -8.8 | 104 | 7 | -18.64 | - | 2.97 |
| Olaparib | 2 | 4.46 | -9.9 | 16 | 15 | -20.86 | 6.9 | 12.18 |
| Levosimendan | 3 | 4.36 | -7.9 | 378 | 19 | -5.69 | 1.3 | 29.56 |
| Ataluren | 4 | 4.36 | -7.9 | 379 | 17 | -10.54 | 4.2 | 24.48 |
| Nifuroxazide | 5 | 4.36 | -7.5 | 583 | 10 | -16.46 | - | 1.75 |
| Fenoterol | 6 | 4.36 | -7.4 | 646 | 4 | -24.41 | - | 6.64 |
| Duvelisib | 7 | 3.93 | -9.1 | 69 | 16 | -14.58 | 5 | 10.41 |
| Carprofen | 8 | 3.83 | -7.7 | 460 | 18 | -6.41 | 2.1 | 22.85 |
| Ergotamine | 9 | 3.45 | -10.5 | 4 | 8 | -17.53 | - | 5.41 |
| Sm13496 | 10 | 3.45 | -10.4 | 6 | 5 | -24.17 | - | 5.22 |
| Raltegravir | 11 | 3.07 | -9.8 | 18 | 14 | -11.62 | 9.2 | 14.09 |
| Abemaciclib | 12 | 3.07 | -9.3 | 46 | 9 | -16.68 | - | 5.21 |
| Fluralaner | 13 | 3.07 | -9.1 | 70 | 1 | -35.56 | - | 3.33 |
| Olmesartan | 14 | 3.07 | -9.1 | 71 | 20 | -13.02 | 0.8 | 13.28 |
| Dantrium | 15 | 2.97 | -7.9 | 380 | 12 | -12.18 | - | 9.55 |
| Tafamidis | 16 | 2.97 | -7.6 | 514 | 11 | -13.38 | - | 7.74 |
| S-adenosyl-methionine | 17 | 2.97 | -7.6 | 515 | 2 | -31.00 | - | 1.43 |
| Tegaserod | 18 | 2.97 | -7.2 | 828 | 3 | -25.34 | - | 3.47 |
| Conivaptan | 19 | 2.59 | 10.6 | 3 | 6 | 19.14 | | 6.92 |
| Eltrombopag | 20 | 2.59 | -10.3 | 7 | 13 | -26.2 | 9.3 | 11.32 |

The column "Rank" recites the ranks of the 20 drugs among 2016 drugs. The column "Highest pose score among the 20 pose scores" recites the highest pose score among the 20 pose scores. The column "Affinity" recites the docking affinity predicted by AutoDock Vina. The column "Affinity rank" recites the rank among 2016 drugs based on the docking affinity. The column "MD rank" recites the rank among the top 20 drugs subject to further 10 ns simulations based on the average binding free energies and drug leaving times. The column "Mean binding free energy" recites the average binding free energy by MM/GBSA method (related information can be found in Kollman, P.A., Massova, I., Reyes, C., Kuhn, B., Huo, S., Chong, L., Lee, M., Lee, T., Duan, Y., Wang, W., Donini, O., Cieplak, P., Srinivasan, J., Case, D.A., Cheatham, T.E., 2000. Calculating Structures and Free Energies of Complex Molecules: Combining Molecular Mechanics and Continuum Models. Accounts of Chemical Research 33, 889-897; and Massova, I., Kollman, P.A., 2000. Combined molecular mechanical and continuum solvent approach (MM-PBSA/GBSA) to predict ligand binding. Perspectives in Drug Discovery and Design 18, 113-135) over the sampled snapshots during the simulations. The column "Drug leaving time" recites the simulation time when the drug dissociated from the target site (the first time point when the distance of the drug to the target site was > 10 Å) and "-" means the drugs did not dissociate during the whole 10 ns simulations. The column "Longest distance to the target site (Å)" recites the longest distance of the drug to the target site sampled during the 10 ns simulations.

The processing unit 101 suggests Fluralaner (-35.56 kcal/mol), Tegaserod (-25.34 kcal/mol), and Fenoterol (-24.41 kcal/mol) based on their top-ranked highest pose scores, favorable mean MM/GBSA binding free energies, and tight adhesion in the binding pocket suggested by the small departure of the drugs from the target site ("longest distance during simulation to the target site") (Methods 2.9). By analyzing the MM/GBSA-revealde binding affinity from MD trajectories, we took top four drugs except for the native substrate SAM -. All three cannot be found in the top 5 of the original docking results with Fluralaner and Tegaserod ranking 13 and 18, respectively, where 2 out of 3 could stably stay within 4 Å from the target site.

Referring to FIG. 1, FIG. 8 and FIG. 10 together, the internal memory 102, 802 and 1002 and the non-volatile memory 103, 803 and 1003 are configured to store programs. The programs may include program code, and the program code includes computer operation instructions. The internal memory 102 and the non-volatile memory 103 provide instructions and data for the processing unit 101, the internal memory 802 and the non-volatile memory 803 provide instructions and data for the processing unit 801, and the internal memory 1002 and the non-volatile memory 1003 provide instructions and data for the processing unit 1001. The processing unit 101 reads the corresponding computer program from the non-volatile memory 103 into the internal memory 102 and then executes the program, the processing unit 801 reads the corresponding computer program from the non-volatile memory 803 into the internal memory 802 and then executes the program, and the processing unit 1001 reads the corresponding computer program from the non-volatile memory 1003 into the internal memory 1002 and then executes the program.

The processing unit 101, 801 and 1001 may be an integrated circuit chip with a signal processing capability. In the implementation process, the methods and steps disclosed in the foregoing embodiments may be implemented through hardware integrated logic circuits or instructions in a form of software in the processing unit 101, 801 and 1001. The processing unit 101, 801 and 1001 may be a general-purpose processor, including a central processing unit (CPU), a tensor processing unit, a digital signal processor (DSP), an application-specific integrated circuit (ASIC), a field-programmable gate array (FPGA), or another programmable logic device, and can implement or execute the methods and steps disclosed in the foregoing embodiments. In some embodiments of the present invention, a computer-readable recording medium storing a program is further provided. The computer-readable recording medium stores at least one instruction. The at least one instruction, when executed by the processing unit 101, 801 and 1001, enables the processing unit 101, 801 and 1001 to performs the steps and methods disclosed in the aforementioned embodiments. Examples of storage media of the computer include, but are not limited to, a phase-change memory (PRAM), a static random access memory (SRAM), a dynamic random access memory (DRAM), another type of random access memory (RAM), a read-only memory (ROM), an electrically erasable programmable read-only memory (EEPROM), a flash memory or another internal memory technology, a compact disc read-only memory (CD-ROM), a digital versatile disc (DVD) or another optical memory, a magnetic tape cassette, a magnetic tape storage or another magnetic storage device, or any other non-transmission medium, which may be used to store information that can be accessed by a computing device. As defined herein, the computer-readable media does not include transitory media such as a modulated data signal and a carri er.

It should be noted that the drug ranking system 100, the comparison system for drug ranking method 800, and the correction system for drug ranking method 1000 can be integrated in a single computer system or be implemented on different computer systems connected to each other by network. The way of implementation is not limited in the present disclosure.

The method for selecting compounds (e.g., from FDA-approved drugs) for use in the treatment of symptoms or disease caused by SARS-CoV-2 is limited to the drug ranking method described herein. Similar, the method for selecting compounds (e.g., from FDA-approved drugs) with anti-SARS-CoV-2 activity is limited to the drug ranking method described herein.

The compounds (or FDA-approved drugs) selected by the drug ranking method described above are not limited for use in the treatment of symptoms or disease caused by SARS-CoV-2. The medical use may include any symptoms or diseases, such as cancer, chronic inflammation, or rare diseases.

The activity to be suppressed or regulated by the compounds (or FDA-approved drugs) selected by the drug ranking method described above is not limited to anti-SARS-CoV-2 activity. The activity to be suppressed or regulated may include other viral activity, fungal activity, parasitic activity, and bacterial activity.

The pharmaceutical composition for use in the treatment of SARS-CoV-2 infection may include administering to a subject in need thereof the pharmaceutical composition, wherein the pharmaceutical composition includes a therapeutically effective amount of at least one drug. The drug may be at least one of fluralaner, tegaserod and fenoterol. A "therapeutically effective amount" is an amount that produces a predetermined pharmacological, therapeutic, or prophylactic result against a symptom or disease.

The pharmaceutical composition for use in inhibiting SARS-CoV-2 activity may include administering to a subject in need thereof a pharmaceutical composition, wherein the pharmaceutical composition includes a therapeutically effective amount of at least one drug. The drug may be at least one of fluralaner, tegaserod and fenoterol.

The pharmaceutical composition for use in the treatment of SARS-CoV-2 infection may include administering to a subject in need thereof a pharmaceutical composition containing a therapeutically effective amount of one or more compounds. The one or more compounds may be selected from drugs ranked top 20 by the drug ranking method of the embodiments described above.

The pharmaceutical composition for use in inhibiting SARS-CoV-2 activity may include administering to a subject in need thereof a pharmaceutical composition, wherein the pharmaceutical composition includes a therapeutically effective amount of at least one drug. The at least one drug may be selected from drugs ranked top 20 by the drug ranking method of the embodiments described above.

In some embodiments, the anti-SARS-CoV-2 agents selected according to the drug ranking method described above may be fluralaner, tegaserod, fenoterol, or any combination thereof.

The pharmaceutical composition may further include a pharmaceutically acceptable carrier that has been commonly used in drug manufacturing. For example, the pharmaceutically acceptable carriers may include one or more of the following agents: emulsifier, suspending agent, decomposer, disintegrating agent, dispersing agent, binding agent, excipient, stabilizing agent, chelating agent, diluent, binding agent, excipient, stabilizing agent, chelating agent, diluent, gelling agent, preservative, wetting agent, lubricant, absorption delaying agent, liposome, and similar substances. The selection and quantity of these carriers can be determined by one of ordinary skill in the art, depending on the actual circumstances.

The pharmaceutical composition may further include the following solvents: water, normal saline, phosphate buffered saline (PBS), aqueous solution containing alcohol, and any other suitable solvents.

The pharmaceutical composition is suitable for administering a subject. Preferably, the subject is a mammal or human.

The pharmaceutical composition is suitable for administering to a human with asthma.

The pharmaceutical composition may further include a therapeutically effective amount of one or more other therapeutic agents. The therapeutic agents may be selected from the group comprised of corticosteroid, anti-inflammatory signal transduction modulator, β-2 adrenoreceptor agonist bronchodilator, anticholinergic, mucolytic agent, other drugs used for treating SARS-CoV-2 infection, and any combination thereof. More specifically, the other drugs used for treating SARS-CoV-2 infection may be selected from the group comprised of nelfinavir, chloroquine, hydroxychloroquine, lopinavir, ritonavir, favipiravir, dexamethasone, camostat mesylate, heparin, baricitinib, and any combination thereof.

In some embodiments, the SARS-CoV-2 may include all dominant strains identified at various periods of time.

In some embodiments, the SARS-CoV-2 has a sequence with about 70% to about 99% (e.g., about 85%, about 90%, or about 95%) similarity to SEQ ID NO: 1.

**Experiment 1: Drug Screening**

Based on the drug ranking results described in Table 4, fenoterol (mean binding free energy being -24.41 kcal/mol and ranked 6th), fluralaner (mean binding free energy being -35.56 kcal/mol and ranked 13th), and tegaserod (mean binding free energy being -25.34 kcal/mol and ranked 18th) were selected.

The IUPAC name and brand name of the three selected drugs are shown in Table 5. Fluralaner is an insecticide used to treat fleas and ticks in animals and recently proposed to treat vector-borne diseases including malaria and Zika fever. Tegaserod is a serotonin-4 (5-HT4) receptor agonist for treatment of irritable bowel syndrome with constipation (IBS-C). Fenoterol is a β-adrenoreceptor agonist sold as an inhaled bronchodilator for treatment of asthma. None of fluralaner, tegaserod, or fenoterol has ever been used, or suggested to be used, for treatment of any diseases associated with viral infection.

Table 5

| Drug | IUPAC name | Brand name |
|---|---|---|
| Fluralaner | 4-[(5R/S)-5-(3,5-Dichlorophenyl)-4,5-dihydro-5-trifluoromethyl-1,2-oxazol-3-yl]-N-[2-oxo-2-(2,2,2-trifluoroethylamino)ethyl]-o-toluamide | Bravecto® |
| Tegaserod | 1-[(E)-(5-methoxy-1H-indol-3-yl)methylideneamino]-2-pentylguanidine | Zelnorm® |
| Fenoterol | 5-[1-hydroxy-2-[1-(4-hydroxyphenyl)propan-2-ylamino]ethyl]benzene-1,3-diol | Berotec® |

**Experiment** 2: Plaque Assay

2-1. The experiment aims to examine the anti- SARS-CoV-2 activity of the test drugs by plaque assays.

2-2. Experiment material

(1) Virus strain: The virus strain was isolated from sputum specimen of a Taiwanese patient infected with severe acute respiratory syndrome coronavirus 2 (SARS-CoV-2). The virus strain was named hCoV-19/Taiwan/NTU13/2020 (hereinafter referred to as NTU13). The accession ID of the virus strain in GISAID database is EPI ISL_422415. The virus strain has a sequence as set forth in SEQ ID NO: 1.
(2) Cell line: African green monkey kidney (Vero E6) cells purchased from American Type Culture Collection (ATCC, USA) and having the ATCC number of CRL-1586™ were used.
(3) Test drugs: The three FDA-approved drugs, fluralaner, tegaserod and fenoterol, as selected by the method described in Experiment 1.
(4) First media: Dulbecco's modified minimal essential medium (DMEM) (Gibco™, Cat. 11965092) containing 10 vol% of fetal bovine serum (FBS) (Gibco, Cat. 10099141) and 1 vol% of antibiotic-antimycotic (Gibco, Cat. 15240-062).
(5) Second media: DMEM containing 2 μg/mL of TPCK-Trypsin (Sigma-Aldrich, T1426).
(6) Third media: Dulbecco's modified minimal essential medium (DMEM) (Gibco™, Cat. 11965092) containing 2 vol% of fetal bovine serum (FBS) (Gibco, Cat. 10099141), 1 vol% of antibiotic-antimycotic (Gibco, Cat. 15240-062), and 1 vol% of methylcellulose (Sigma-Aldrich, M0387).
(7) Nelfinavir: A SARS-CoV-2 3CL protease inhibitor sold under the brand name of Viracept®.

2-3. Experiment procedure

A. Cell culture

The day before infection, Vero E6 cells were seeded to a 6-well culture plate in the first media and incubated overnight.

B. Virus culture

Vero E6 cells were infected with NTU13 and incubated in the second media for 1 h at 37 oC, to allow the virus to propagate in the cells (50-100 plaque forming unit, PFU). After removal of virus inoculum, the Vero E6 cells were rinsed once with PBS and incubated in the third media shown in Table 6 at 37 oC. Supernatant of the culture was harvested when over 70% of the Vero E6 cells exhibited cytopathic effects (CPE). Thereafter, the viral titers were determined by plaque assays. In the experiment, the cells were divided into seven groups, including one positive control group (PCG), two negative control groups (NCG1 and NCG2), and four experiment groups (EG0, EG1, EG2, and EG3).

Table 6

| Groups | | Media |
|---|---|---|
| Positive control group (PCG) | | Third media containing 3.6 μM of nelfinavir |
| Negative control group | negative control group 1 (NCG1) | Third media without any test drug |
| | negative control group 2 (NCG2) | Third media containing 0.5 vol% of DMSO |
| Experiment group | experiment group 0 (EG0) | Third media containing 10 μM of fluralaner |
| | experiment group 1 (EG1) | Third media containing 40 μM of fenoterol |
| | experiment group 2 (EG2) | Third media containing 10 μM of fenoterol |
| | experiment group 3 (EG3) | Third media containing 10 μM of tegaserod |

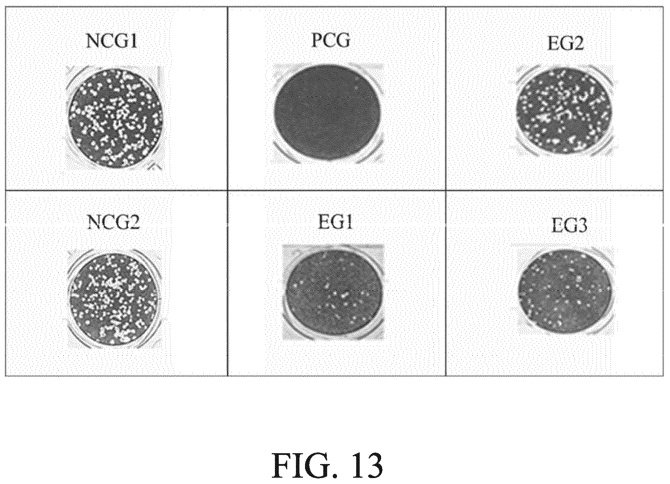

After 5 days of incubation, the third media was removed from the culture. The Vero E6 cells were fixed with 10% formalin (Sigma-Aldrich, HT501128) overnight. After removal of the formalin, the Vero E6 cells were stained with 0.5% crystal violet (VWR life science, 0528), and the resulting plaques (shown as the white dots in FIG. 13) were counted. The percentage of suppression was calculated by the equation of [1-(VD/VC)]x100%, where VD and VC refer to the virus titer in the presence and absence of the test drugs, respectively. The minimal concentrations of drugs required to reduce 50% of plaque counts (EC50) were determined by regression analysis of the dose-response curves generated from the plaque assay. For each data point, the measurements were repeated at least in triplicates to obtain the average value, standard deviations, and standard errors.

2-4. Experiment results

FIG.13 shows the staining results of the plaque assay for determining the anti-SARS-CoV-2 activity of the test drugs selected by the drug ranking method of the embodiments of the present disclosure. As shown in FIG. 13, significant plaque reduction was observed in experiment group 1 (EG1), experiment group 2 (EG2), and experiment group 3 (EG3), as compared to the negative control groups (NCG1 and NCG2). Specifically, EG1 and EG2 showed that at least 10 $\mu$M, more preferably 40 $\mu$M, of fenoterol was effective in suppressing SARS-CoV-2 replication. EG3 showed that at least 10 $\mu$M of tegaserod was effective in suppression SARS-CoV-2 replication. It is to be noted that negative control group 1 (NCG1) and negative control group 2 (NG2) happened to result in the same average number of plaques.

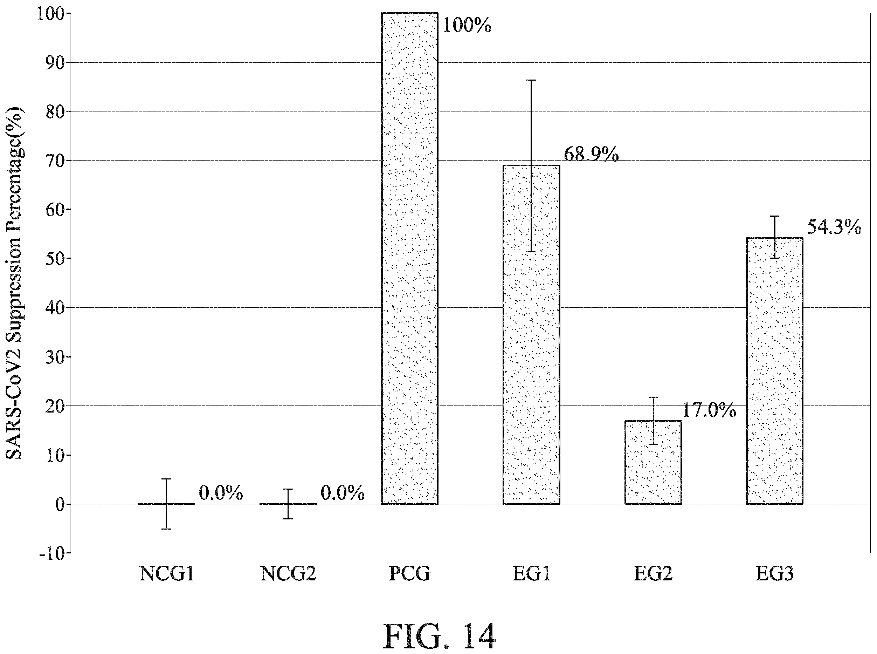

FIG. 14 is a histogram showing the anti-SARS-CoV-2 activity of the test drugs as calculated by quantification of the plaque assay results in FIG. 13. As shown in FIG. 14, EG1 and EG3 showed significant anti-SARS-CoV-2 activity. Specifically, the average percentage of suppression of SARS-CoV-2 replication in experiment group 1 (EG1) was 68.9%, meaning that the plaque count was reduced by about 70% by 40 $\mu$M of fenoterol. The average percentage of suppression of SARS-CoV-2 replication in experiment group 2 (EG2, treated by 10 $\mu$M of fenoterol) was 17.0%. The average percentage of SARS-CoV-2 suppression in experiment group 3 (EG3) was 54.3%, meaning that the plaque count was reduced by about 54% by 10 $\mu$M of tegaserod. It is to be noted that 10 $\mu$M fluralaner was shown to be toxic to Vero E6 cells, therefore the anti-SARS-CoV-2 activity of the experiment group 0 (EG0, treated by 10 $\mu$M of fluralaner) is not shown in FIG. 14.

Still referring to FIG. 14, the minimal concentrations of compounds required to reduce 50% of plaque count (EC50) (i.e., 50% suppression along the y-axis in FIG. 14) were calculated by regression analysis of the dose-response curves generated from the plaque assays. The results suggested that EC50 of fenoterol for SARS-CoV-2 infected Vero E6 cells was 30 $\mu$M, and that EC50 of tegaserod for SARS-CoV-2 infected Vero E6 cells was slightly lower than 10 $\mu$M.

Accordingly, the results described above demonstrated that tegaserod and fenoterol are promising candidates for treating SARS-CoV-2 infection. Furthermore, while there is no apparent evidence indicating any increased risks of SARS-CoV-2 (COVID-19) infection or death in patients with asthma, significant suppression of SARS-CoV-2 activity by fenoterol suggests that fenoterol could be used as a timely reliever for patients with asthma after SARS-CoV-2 infection.

Based on the above, in some embodiments of the instant disclosure, a drug ranking method, a drug ranking system, a comparison method for drug ranking method, a comparison system for drug ranking method, a correction method for drug ranking method, a correction system for drug ranking method, a computer readable recording medium with a stored program, and a non-transitory computer program product are provided. Hence, through the consideration of at least one feature of the pose, an accurate drug ranking method is provided. Furthermore, a comparison method for drug ranking method and a correction method for drug ranking method are also provided. In some embodiments of the instant disclosure, a method of selecting drugs that could be repurposed for treating diseases or symptoms (e.g., those caused by SARS-CoV-2 infection) other than their known indications.

While the instant disclosure has been described by the way of example and in terms of the preferred embodiments, it is to be understood that the invention need not be limited to the disclosed embodiments.

**Claims**

**1.** A drug ranking method executed by a processing unit (101), wherein the drug ranking method is configured to rank a plurality of drugs according to a protein target; the drug ranking method comprises:

(a) according to a plurality of benchmark drugs from the plurality of drugs, a plurality of benchmark drug target proteins corresponding to the benchmark drugs, and a plurality of true binding poses and a plurality of decoy poses generated by docking the drugs with the benchmark drug target proteins, obtaining a statistics distribution pair for each of at least one feature, wherein the statistics distribution pair of each of the at least one feature comprises a true binding pose statistics distribution and a decoy pose statistics distribution;

(b) docking the protein target for each of the drugs to obtain a plurality of poses (201) and at least one feature value for each of the poses (201), wherein the at least one feature value corresponds to the at least one feature;

(c) according to the at least one feature value of each of the poses (201) and the statistics distribution pair of each of the at least one feature, obtaining a score of each of the poses (201) for each of the drugs so as to obtain a plurality of pose scores of each of the drugs; and

(d) ranking the drugs according to the pose scores of each of the drugs;

wherein the at least one feature comprises a distance between one of the poses (201) and at least one active site residue, wherein the distance between one of the poses (201) and the at least one active site residue is a distance between a center of mass of heavy atoms of one of the drugs and a center of mass of heavy atoms of all the active site residues,

wherein the active site residues are the residues that perform one of the following functions: 1. direct involvement in the catalytic mechanism; 2. exerting an effect on another residue or water molecule which is directly involved in the catalytic mechanism which aids catalysis; 3. stabilization of a proposed transition-state intermediate; and 4. exerting an effect on a substrate or cofactor which aids catalysis; and the residues have a conservation score greater than or equal to 7 as calculated by Consurf Database.

2. The drug ranking method according to claim 1, wherein the at least one feature comprises a binding affinity, a distance between a pose (201) and an assigned target residue (202), and a size of a pose cluster.

3. The drug ranking method according to claim 1, wherein the step (a) comprises:

(a1) executing following steps for a current feature of the at least one feature:

(a11) according to a plurality of first feature values of the true binding poses corresponding to the current feature, in a plurality of first value intervals, calculating a first ratio of the true binding poses in each of the first value intervals with respect to the true binding poses in all of the first value intervals to obtain a preliminary true binding pose statistics distribution (301, 303, 305, 307, 401, 403, 405, 407, 409, 411) corresponding to the current feature;

(a12) according to a plurality of second feature values of the decoy poses corresponding to the current feature, in a plurality of second value intervals, calculating a second ratio of the decoy poses in each of the second value intervals with respect to the decoy poses in all of the second value intervals to obtain a preliminary decoy pose statistics distribution (302, 304, 306, 308, 402, 404, 406, 408, 410, 412) corresponding to the current feature;

(a13) fitting the preliminary true binding pose statistics distribution (301, 303, 305, 307, 401, 403, 405, 407, 409, 411) corresponding to the current feature by a first fitting function to obtain the true binding pose statistics distribution corresponding to the current feature; fitting the preliminary decoy pose statistics distribution (302, 304, 306, 308, 402, 404, 406, 408, 410, 412) corresponding to the current feature by a second fitting function to obtain the decoy pose statistics distribution corresponding to the current feature: and

(a2) repeatedly executing the step (a1) until all the at least one feature is processed.

4. The drug ranking method according to claim 1, wherein the step (c) comprises: executing following steps for a current pose of the poses (201):

(c1) executing following steps for a current feature of the at least one feature:

(c11) applying a current feature value corresponding to the current feature in the at least one feature value into the true binding pose statistics distribution corresponding to the current feature so as to obtain a first probability;

(c12) applying the current feature value corresponding to the current feature in the at least one feature value into the decoy pose statistics distribution corresponding to the current feature so as to obtain a second probability; and

(c13) dividing the first probability by the second probability to obtain a value and deriving the logarithm of the value so as to obtain a current feature score corresponding to the current feature;

(c2) repeatedly executing the step (c1) until all the at least one feature is processed; and

(c3) after all the at least one feature is processed, summing up all the current features scores obtained from the steps (c1)-(c2) so as to obtain the score of the current pose.

5. A drug ranking system, wherein the drug ranking system comprises a processing unit (101), and the processing unit

EP 4 120 277 B1

(101) is configured to execute following steps according to a protein target to rank a plurality of drugs:

(a) according to a plurality of benchmark drugs from the plurality of drugs, a plurality of benchmark drug target proteins corresponding to the benchmark drugs, and a plurality of true binding poses and a plurality of decoy poses generated by docking the drugs with the benchmark drug target proteins, obtaining a statistics distribution pair for each of at least one feature, wherein the statistics distribution pair of each of the at least one feature comprises a true binding pose statistics distribution and a decoy pose statistics distribution;

(b) docking the protein target for each of the drugs to obtain a plurality of poses (201) and at least one feature value for each of the poses (201), wherein the at least one feature value corresponds to the at least one feature;

(c) according to the at least one feature value of each of the poses (201) and the statistics distribution pair of each of the at least one feature, obtaining a score of each of the poses (201) for each of the drugs so as to obtain a plurality of pose scores of each of the drugs; and

(d) ranking the drugs according to the pose scores of each of the drugs,

wherein the at least one feature comprises a distance between one of the poses (201) and at least one active site residue, wherein the distance between one of the pose (201) and the at least one active site residue is a distance between a center of mass of heavy atoms of one of the drugs and a center of mass of heavy atoms of all the active site residues,

wherein the active site residues are the residues that perform one of the following functions: 1. direct involvement in the catalytic mechanism; 2. exerting an effect on another residue or water molecule which is directly involved in the catalytic mechanism which aids catalysis; 3. stabilization of a proposed transition-state intermediate; and 4. exerting an effect on a substrate or cofactor which aids catalysis; and the residues have a conservation score greater than or equal to 7 as calculated by Consurf Database.

6. The drug ranking system according to claim 5, wherein the at least one feature comprises a binding affinity, a distance between a pose (201) and an assigned target residue (202), and a size of a pose cluster.

7. The drug ranking system according to claim 5, wherein the processing unit (101) performs MD simulations for user-selected poses, and based on average binding free energies and drug leaving times during the MD simulations, ranks top-ranked 20 drugs in the step (d).

**Patentansprüche**

1. Ein Verfahren zum Ranking von Arzneimitteln, das von einer Verarbeitungseinheit (101) ausgeführt wird, wobei das Verfahren zum Ranking von Arzneimitteln so konfiguriert ist, dass es eine Vielzahl von Arzneimitteln entsprechend einem Zielprotein in eine Rangfolge bringt; das Verfahren zur Rangfolge von Arzneimitteln umfasst:

(a) gemäß einer Vielzahl von Referenz-Arzneimitteln aus der Vielzahl von Arzneimitteln, einer Vielzahl von Referenz-Zielproteinen für Arzneimittel, die zu den Referenz-Arzneimitteln gehören, und einer Vielzahl von echten Bindungspositionen und einer Vielzahl von Scheinpositionen, die durch Andocken der Arzneimittel an die Referenz-Zielproteinen für Arzneimittel erzeugt werden, Erhalten eines Statistikverteilungspaares für jedes von mindestens einem Merkmal, wobei das Statistikverteilungspaar jedes der mindestens einen Merkmale eine Statistikverteilung der echten Bindungsposition und eine Statistikverteilung der Scheinposition umfasst;

(b) Andocken des Protein-Ziels für jedes der Arzneimittel, um eine Vielzahl von Positionen (201) und mindestens einen Merkmalswert für jede der Positionen (201) zu erhalten, wobei der mindestens eine Merkmalswert dem mindestens einen Merkmal entspricht;

(c) Erhalten einer Punktzahl für jede der Positionen (201) für jedes der Arzneimittel gemäß dem mindestens einen Merkmalswert jeder der Positionen (201) und dem Statistikverteilungspaar jedes der mindestens einen Merkmale, um eine Vielzahl von Positionspunktzahlen für jedes der Arzneimittel zu erhalten; und

(d) Ordnen der Arzneimittel gemäß den Positions-Bewertungen jedes der Arzneimittel;

wobei das mindestens eine Merkmal einen Abstand zwischen einer der Positionen (201) und mindestens einem Rest der aktiven Stelle umfasst, wobei der Abstand zwischen einer der Positionen (201) und dem mindestens einen Rest der aktiven Stelle ein Abstand zwischen einem Schwerpunkt schwerer Atome eines der Arzneimittel und einem Schwerpunkt schwerer Atome aller Reste der aktiven Stelle ist,

wobei die Reste der aktiven Stelle die Reste sind, die eine der folgenden Funktionen erfüllen:

1. direkte Beteiligung am katalytischen Mechanismus; 2. Ausübung einer Wirkung auf einen anderen Rest oder ein Wassermolekül, das direkt am katalytischen Mechanismus beteiligt ist, der die Katalyse unterstützt; 3. Stabilisierung eines vorgeschlagenen Übergangszustands-Zwischenprodukts; und 4. Ausübung einer Wirkung auf ein Substrat oder einen Cofaktor, der die Katalyse unterstützt; und die Reste haben einen Konservierungswert von größer oder gleich 7, berechnet anhand der Consurf-Datenbank.

**2.** Das Verfahren zum Ranking von Arzneimitteln gemäß Anspruch 1, wobei das mindestens eine Merkmal eine Bindungsaffinität, einen Abstand zwischen einer Position (201) und einem zugewiesenen Zielrest (202) und eine Größe eines Positions-Clusters umfasst.

**3.** Verfahren zum Ranking von Arzneimitteln gemäß Anspruch 1, wobei der Schritt (a) umfasst:

(al) Ausführen der folgenden Schritte für ein aktuelles Merkmal des mindestens einen Merkmals:

(a11) gemäß einer Vielzahl von ersten Merkmalswerten der tatsächlichen Bindungspositionen, die dem aktuellen Merkmal entsprechen, in einer Vielzahl von ersten Werteintervallen, Berechnen eines ersten Verhältnisses der tatsächlichen Bindungspositionen in jedem der ersten Werteintervalle in Bezug auf die tatsächlichen Bindungspositionen in allen ersten Werteintervallen, um eine vorläufige Statistikverteilung der tatsächlichen Bindungspositionen (301, 303, 305, 307, 401, 403, 405, 407, 409, 411) zu erhalten, die dem aktuellen Merkmal entspricht;
(a12) gemäß einer Vielzahl von zweiten Merkmalswerten der Köderpositionen, die dem aktuellen Merkmal entsprechen, in einer Vielzahl von zweiten Werteintervallen, Berechnen eines zweiten Verhältnisses der Köderpositionen in jedem der zweiten Werteintervalle in Bezug auf die Köderpositionen in allen zweiten Werteintervallen, um eine vorläufige Köderpositionen-Statistikverteilung (302, 304, 306, 308, 402, 404, 406, 408, 410, 412) zu erhalten, die dem aktuellen Merkmal entspricht;

(a13) Anpassen der vorläufigen Verteilung der statistischen Daten zur tatsächlichen Bindungsposition (301, 303, 305, 307, 401, 403, 405, 407, 409, 411) entsprechend dem aktuellen Merkmal durch eine erste Anpassungsfunktion, um die Statistikverteilung der tatsächlichen Bindungsposition entsprechend dem aktuellen Merkmal zu erhalten;
Anpassen der vorläufigen Statistikverteilung der Scheinposition (302, 304, 306, 308, 402, 404, 406, 408, 410, 412) entsprechend dem aktuellen Merkmal durch eine zweite Anpassungsfunktion, um die Köder-Positionen-Statistikverteilung entsprechend dem aktuellen Merkmal zu erhalten; und
(a2) wiederholtes Ausführen des Schritts (a1), bis alle mindestens ein Merkmal verarbeitet sind.

**4.** Verfahren zum Ranking von Arzneimitteln gemäß Anspruch 1, wobei der Schritt (c) umfasst: Ausführen der folgenden Schritte für eine aktuelle Pose der Posen (201):

(c1) Ausführen der folgenden Schritte für ein aktuelles Merkmal des mindestens einen Merkmals:

(c11) Anwenden eines aktuellen Merkmalswerts, der dem aktuellen Merkmal in dem mindestens einen Merkmalswert entspricht, auf die Verteilung der wahren Bindungspositionen-Statistik, die dem aktuellen Merkmal entspricht, um eine erste Wahrscheinlichkeit zu erhalten;
(c12) Anwenden des aktuellen Merkmalswerts, der dem aktuellen Merkmal in dem mindestens einen Merkmalswert entspricht, auf die dem aktuellen Merkmal entsprechende Statistikverteilung der Scheinpositionen, um eine zweite Wahrscheinlichkeit zu erhalten; und
(c13) Dividieren der ersten Wahrscheinlichkeit durch die zweite Wahrscheinlichkeit, um einen Wert zu erhalten, und Ableiten des Logarithmus des Werts, um eine aktuelle Merkmalsbewertung zu erhalten, die dem aktuellen Merkmal entspricht;

(c2) wiederholtes Ausführen des Schritts (c1), bis alle der mindestens einen Merkmale verarbeitet sind; und
(c3) nachdem alle der mindestens einen Merkmale verarbeitet sind, Summieren aller aktuellen Merkmalswerte, die aus den Schritten (c1)-(c2) erhalten wurden, um den Wert der aktuellen Positionen zu erhalten.

**5.** Ein System zum Ranking von Arzneimitteln, wobei das Arzneimittel-Rankingsystem eine Verarbeitungseinheit (101) umfasst und die Verarbeitungseinheit (101) so konfiguriert ist, dass sie die folgenden Schritte entsprechend einem Zielprotein ausführt, um eine Vielzahl von Arzneimitteln zu bewerten:

(a) gemäß einer Vielzahl von Referenz-Arzneimitteln aus der Vielzahl von Arzneimitteln, einer Vielzahl von Zielproteinen für ReferenzArzneimittel, die den Referenz-Arzneimitteln entsprechen, und einer Vielzahl von echten Bindungspositionen und einer Vielzahl von Scheinpositionen, die durch Andocken der Arzneimittel an die Zielproteine für Referenz-Arzneimittel erzeugt werden, Erhalten eines Statistikverteilungspaares für jedes von mindestens einem Merkmal, wobei das Statistikverteilungspaar jedes der mindestens einen Merkmale eine Statistikverteilung der echten Bindungsposition und eine Statistikverteilung der Scheinposition umfasst;

(b) Andocken des Protein-Targets für jedes der Arzneimittel, um eine Vielzahl von Positionen (201) und mindestens einen Merkmalswert für jede der Positionen (201) zu erhalten, wobei der mindestens eine Merkmalswert dem mindestens einen Merkmal entspricht;

(c) basierend auf dem mindestens einen Merkmalswert jeder der Positionen (201) und dem Statistikverteilungspaar jedes der mindestens einen Merkmale, Ermitteln einer Punktzahl jeder der Positionen (201) für jedes der Arzneimittel, um eine Vielzahl von Positions-Punktzahlen jedes der Arzneimittel zu erhalten; und

(d) Ranken der Arzneimittel gemäß den Positions-Bewertungen jedes der Arzneimittel,

wobei das mindestens eine Merkmal einen Abstand zwischen einer der Posen (201) und mindestens einem Rest der aktiven Stelle umfasst, wobei der Abstand zwischen einer der Posen (201) und dem mindestens einen Rest der aktiven Stelle ein Abstand zwischen einem Schwerpunkt schwerer Atome eines der Arzneimittel und einem Schwerpunkt schwerer Atome aller Reste der aktiven Stelle ist,

wobei die aktiven Stellenreste die Reste sind, die eine der folgenden Funktionen erfüllen:

1. direkte Beteiligung am katalytischen Mechanismus; 2. Ausübung einer Wirkung auf einen anderen Rest oder ein Wassermolekül, das direkt am katalytischen Mechanismus beteiligt ist, der die Katalyse unterstützt; 3. Stabilisierung eines vorgeschlagenen Übergangszustands-Zwischenprodukts; und 4. Ausübung einer Wirkung auf ein Substrat oder einen Cofaktor, der die Katalyse unterstützt; und die Reste haben einen Konservierungswert von größer oder gleich 7, berechnet anhand der Consurf-Datenbank.

6.  Das Arzneimittel-Ranking-System gemäß Anspruch 5, wobei das mindestens eine Merkmal eine Bindungsaffinität, einen Abstand zwischen einer Position (201) und einem zugewiesenen Zielrest (202) und eine Größe eines Positions-Clusters umfasst.

7.  Das Arzneimittel-Ranking-System gemäß Anspruch 5, wobei die Verarbeitungseinheit (101) MD-Simulationen für vom Benutzer ausgewählte Positionen durchführt und auf der Grundlage der durchschnittlichen freien Bindungsenergien und Arzneimittelabgangszeiten während der MD-Simulationen die 20 bestbewerteten Arzneimittel in Schritt (d) bewertet.

**Revendications**

1.  Procédé de classement de médicaments exécuté par une unité de traitement (101), dans lequel le procédé de classement de médicaments est configuré pour classer une pluralité de médicaments en fonction d'une protéine cible ; le procédé de classement de médicaments comprend :

    (a) en fonction d'une pluralité de médicaments de référence parmi la pluralité de médicaments, d'une pluralité de protéines cibles de médicaments de référence correspondant aux médicaments de référence, et d'une pluralité de poses de liaison réelles et d'une pluralité de poses leurres générées par l'ancrage des médicaments aux protéines cibles de médicaments de référence, l'obtention d'une paire de distributions statistiques pour chacune d'au moins une caractéristique, dans laquelle la paire de distributions statistiques de chacune d'au moins une caractéristique comprend une distribution statistique de poses de liaison réelles et une distribution statistique de poses leurres ;

    (b) ancrer la protéine cible pour chacun des médicaments afin d'obtenir une pluralité de poses (201) et au moins une valeur caractéristique pour chacune des poses (201), dans laquelle la au moins une valeur caractéristique correspond à la au moins une caractéristique ;

    (c) en fonction de la au moins une valeur caractéristique de chacune des poses (201) et de la paire de distribution statistique de chacune des au moins une caractéristique, obtenir un score de chacune des poses (201) pour chacun des médicaments afin d'obtenir une pluralité de scores de pose de chacun des médicaments ; et

    (d) classer les médicaments en fonction des scores de pose de chacun des médicaments ;

    dans lequel la au moins une caractéristique comprend une distance entre l'une des poses (201) et au moins un résidu du site actif, dans lequel la distance entre l'une des poses (201) et le au moins un résidu du site actif est une distance entre un centre de masse d'atomes lourds de l'un des médicaments et un centre de masse d'atomes lourds de tous les résidus du site actif,

    dans lequel les résidus du site actif sont les résidus qui remplissent l'une des fonctions suivantes :

    1. participation directe au mécanisme catalytique ; 2. exercice d'un effet sur un autre résidu ou une molécule d'eau directement impliqué dans le mécanisme catalytique qui facilite la catalyse ; 3. stabilisation d'un intermédiaire d'état de transition proposé ; et 4. exercice d'un effet sur un substrat ou un cofacteur qui facilite la catalyse ; et les résidus ont un score de conservation supérieur ou égal à 7, tel que calculé par la base de données Consurf.

2. Procédé de classement de médicaments selon la revendication 1, dans lequel la au moins une caractéristique comprend une affinité de liaison, une distance entre une pose (201) et un résidu cible attribué (202), et une taille d'un groupe de poses.

3. Procédé de classement de médicaments selon la revendication 1, dans lequel l'étape (a) comprend :

(a1) l'exécution des étapes suivantes pour une caractéristique actuelle parmi au moins une caractéristique :

(a11) en fonction d'une pluralité de premières valeurs caractéristiques des poses de liaison réelles correspondant à la caractéristique actuelle, dans une pluralité de premiers intervalles de valeurs, calculer un premier rapport entre les poses de liaison réelles dans chacun des premiers intervalles de valeurs par rapport aux poses de liaison réelles dans tous les premiers intervalles de valeurs afin d'obtenir une distribution statistique préliminaire des poses de liaison réelles (301, 303, 305, 307, 401, 403, 405, 407, 409, 411) correspondant à la caractéristique actuelle ;

(a12) selon une pluralité de secondes valeurs caractéristiques des poses leurres correspondant à la caractéristique actuelle, dans une pluralité de seconds intervalles de valeurs, calculer un second rapport des poses leurres dans chacun des seconds intervalles de valeurs par rapport aux poses leurres dans tous les seconds intervalles de valeurs afin d'obtenir une distribution statistique préliminaire des poses leurres (302, 304, 306, 308, 402, 404, 406, 408, 410, 412) correspondant à la caractéristique actuelle ;

(a13) ajuster la distribution statistique préliminaire des poses de liaison réelles (301, 303, 305, 307, 401, 403, 405, 407, 409, 411) correspondant à la caractéristique actuelle à l'aide d'une première fonction d'ajustement afin d'obtenir la distribution statistique des poses de liaison réelles correspondant à la caractéristique actuelle ; ajuster la distribution statistique préliminaire des poses leurres (302, 304, 306, 308, 402, 404, 406, 408, 410, 412) correspondant à la caractéristique actuelle à l'aide d'une deuxième fonction d'ajustement afin d'obtenir la distribution statistique de la pose leurre correspondant à la caractéristique actuelle ; et

(a2) exécuter de manière répétée l'étape (a1) jusqu'à ce que toutes les caractéristiques soient traitées.

4. Procédé de classement de médicaments selon la revendication 1, dans lequel l'étape (c) comprend : l'exécution des étapes suivantes pour une pose actuelle parmi les poses (201) :

(c1) exécuter les étapes suivantes pour une caractéristique actuelle parmi les caractéristiques:

(c11) appliquer une valeur de caractéristique actuelle correspondant à la caractéristique actuelle dans au moins une valeur de caractéristique dans la distribution statistique de la pose de liaison réelle correspondant à la caractéristique actuelle afin d'obtenir une première probabilité ;

(c12) appliquer la valeur de caractéristique actuelle correspondant à la caractéristique actuelle dans la au moins une valeur de caractéristique dans la distribution statistique de pose leurre correspondant à la caractéristique actuelle afin d'obtenir une deuxième probabilité ; et

(c13) diviser la première probabilité par la deuxième probabilité pour obtenir une valeur et dériver le logarithme de la valeur afin d'obtenir un score de caractéristique actuel correspondant à la caractéristique actuelle ;

(c2) exécuter de manière répétée l'étape (c1) jusqu'à ce que toutes les caractéristiques soient traitées ; et

(c3) après le traitement de toutes les caractéristiques, additionner tous les scores de caractéristiques actuels obtenus à partir des étapes (c1) à (c2) afin d'obtenir le score de la pose actuelle.

5. Système de classement de médicaments, dans lequel le système de classement de médicaments comprend une unité de traitement (101), et l'unité de traitement (101) est configurée pour exécuter les étapes suivantes en fonction d'une protéine cible afin de classer une pluralité de médicaments :

(a) en fonction d'une pluralité de médicaments de référence parmi la pluralité de médicaments, d'une pluralité de protéines cibles de référence correspondant aux médicaments de référence, et d'une pluralité de poses de liaison réelles et d'une pluralité de poses leurres générées par l'ancrage des médicaments aux protéines cibles de référence, obtenir une paire de distributions statistiques pour chacune d'au moins une caractéristique, la paire de distributions statistiques de chacune d'au moins une caractéristique comprenant une distribution statistique des poses de liaison réelles et une distribution statistique des poses leurres ;

(b) amarrage de la protéine cible pour chacun des médicaments afin d'obtenir une pluralité de poses (201) et au moins une valeur de caractéristique pour chacune des poses (201), dans lequel la au moins une valeur de caractéristique correspond à la au moins une caractéristique ;

(c) en fonction de la valeur d'au moins une caractéristique de chacune des poses (201) et de la paire de distribution statistique de chacune des au moins une caractéristique, obtenir score de chacune des poses (201) pour chacun des médicaments afin d'obtenir une pluralité de scores de pose de chacun des médicaments ; et

(d) classer les médicaments en fonction des scores de pose de chacun des médicaments, dans lequel la au moins une caractéristique comprend une distance entre l'une des poses (201) et au moins un résidu du site actif, dans lequel la distance entre l'une des poses (201) et le au moins un résidu du site actif est une distance entre un centre de masse d'atomes lourds de l'un des médicaments et un centre de masse d'atomes lourds de tous les résidus du site actif,

dans lequel les résidus du site actif sont les résidus qui remplissent l'une des fonctions suivantes : 1. participation directe au mécanisme catalytique ; 2. exercice d'un effet sur un autre résidu ou une molécule d'eau directement impliqué dans le mécanisme catalytique qui facilite la catalyse ; 3. stabilisation d'un intermédiaire d'état de transition proposé ; et 4. exercice d'un effet sur un substrat ou un cofacteur qui facilite la catalyse ; et les résidus ont un score de conservation supérieur ou égal à 7, tel que calculé par la base de données Consurf.

6. Système de classement des médicaments selon la revendication 5, dans lequel la au moins une caractéristique comprend une affinité de liaison, une distance entre une pose (201) et un résidu cible attribué (202), et une taille d'un groupe de poses.

7. Système de classement des médicaments selon la revendication 5, dans lequel l'unité de traitement (101) effectue des simulations MD pour les poses sélectionnées par l'utilisateur et, sur la base des énergies libres de liaison moyennes et des temps de sortie des médicaments pendant les simulations MD, classe les 20 médicaments les mieux classés à l'étape (d).

EP 4 120 277 B1

FIG. 1

FIG. 2

FIG. 3A

FIG. 3B

FIG. 3C

FIG. 3D

FIG. 4A

FIG. 4B

FIG. 4C

According to a plurality of benchmark drugs in the drugs, a plurality of benchmark drug target proteins corresponding to the benchmark drugs, and a plurality of true binding poses and a plurality of decoy poses generated by docking the drugs with the benchmark drug target proteins, obtaining a statistics distribution pair for each of at least one feature, wherein the statistics distribution pair of each of the at least one feature comprises a true binding pose statistics distribution and a decoy pose statistics distribution — S501

Docking the protein target for each of the drugs to obtain a plurality of poses and at least one feature value for each of the poses, wherein the at least one feature value corresponds to the at least one feature — S502

According to the at least one feature value of each of the poses and the statistics distribution pair of each of the at least one feature, obtaining a score of each of the poses for each of the drugs so as to obtain a plurality of pose scores of each of the drugs — S503

Ranking the drugs according to the pose scores of each of the drugs — S504

# FIG. 5

S601

According to a plurality of first feature values of the true binding poses corresponding to the current feature, in a plurality of first value intervals, calculating a first ratio of the true binding poses in each of the first value intervals to obtain a preliminary true binding pose statistics distribution corresponding to the current feature

S6011

According to a plurality of second feature values of the decoy poses corresponding to the current feature, in a plurality of second value intervals, calculating a second ratio of the decoy poses in each of the second value intervals to obtain a preliminary decoy pose statistics distribution corresponding to the current feature

S6012

Fitting the preliminary true binding pose statistics distribution corresponding to the current feature by a first fitting function to obtain the true binding pose statistics distribution corresponding to the current feature; fitting the preliminary decoy pose statistics distribution corresponding to the current feature by a second fitting function to obtain the decoy pose statistics distribution corresponding to the current feature

S6013

No — Is all the at least one feature processed?

S602

Yes

FIG. 6

Applying a current feature value corresponding to the current feature in the at least one feature value into the true binding pose statistics distribution corresponding to the current feature so as to obtain a first probability — S7011

Applying the current feature value corresponding to the current feature in the at least one feature value into the decoy pose statistics distribution corresponding to the current feature so as to obtain a second probability — S7012

Dividing the first probability by the second probability to obtain a value and deriving the logarithm of the value so as to obtain a current feature score corresponding to the current feature — S7013

S701

No — Is All the at least one feature processed? — S702

Yes

After all the at least one feature is processed, summing up all the current features scores obtained from the steps S701-S702 so as to obtain the score of the current pose — S703

FIG. 7

FIG. 8

Obtaining a plurality of
benchmark drugs from the drugs — S901

S902

Executing following steps for a current benchmark
drug of the benchmark drugs: according to a
current benchmark drug target protein
corresponding to the current benchmark drug,
ranking the drugs to obtain a ranking by the current
drug ranking method; and obtaining a drug ranking
of the current benchmark drug according to the
ranking — S9021

S9022

No — Are all the benchmark
drugs processed?

Yes

After all the benchmark drugs are processed,
averaging the rankings obtained from the step
S9021-S9022 so as to obtain a drug ranking
method score of the current drug ranking method — S9023

According to the drug ranking method score of
each of the drug ranking methods, comparing the
drug ranking methods with each other so as to
obtain a drug ranking method ranking for the
drug ranking methods — S903

FIG. 9

FIG. 10

Obtaining a plurality of
benchmark drugs from the drugs ⟶S1101

Adjusting the at least one adjustable parameter of
the drug ranking method so as to optimize a drug ⟶S1102
ranking method score

# FIG. 11

According to a current benchmark drug target
protein corresponding to the current benchmark
drug, ranking the drugs to obtain a ranking by the
current drug ranking method; and obtaining a drug ⟶S1201
ranking of the current benchmark drug according to
the ranking

S1202

No ◄ Are all the benchmark
drugs processed?

Yes

Averaging the rankings obtained from the step
S1201-S1202 so as to obtain the drug ranking ⟶S1203
method score

# FIG. 12

FIG. 13

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Fenoterol and dobutamine as SARS-CoV-2 main protease inhibitors: A virtual screening study. **BO-LELLI KAYHAN et al.** JOURNAL OF MOLECULAR STRUCTURE. ELSEVIER, vol. 1228, 13
- **BERMAN, H.M** ; **WESTBROOK J** ; **FENG Z.** ; **GILLILAND G** ; **BHAT T.N** ; **WEISSIG H** ; **SHINDYA-LOV I.N** ; **BOURNE P.E**. The Protein Data Bank.. *Nucleic Acids Research*, 2000, vol. 28, 235-242
- **WESTBROOK, J.D.** ; **BURLEY, S.K**. How Structural Biologists and the Protein Data Bank Contributed to Recent FDA New Drug Approvals.. *Structure*, 2019, vol. 27, 211-217
- **BEN CHORIN A.** ; **MASRATI G** ; **KESSEL A** ; **NARUNSKY A.** ; **SPRINZAK J** ; **LAHAV S.** ; **ASHKENAZY H** ; **BEN-TAL N**. ConSurf-DB: An accessible repository for the evolutionary conservation patterns of the majority of PDB proteins.. *Protein Science*, 2020, vol. 29, 25-267
- **GAIL J. BARTLETT** ; **CRAIG T. PORTER** ; **NEERA BORKAKOTI** ; **JANET M. THORNTON**. Analysis of Catalytic Residues in Enzyme Active Sites. *Journal of Molecular Biology*, 2002, vol. 324 (1), 105-121
- **HUANG, D** ; **CAFLISCH, A.** Small Molecule Binding to Proteins: Affinity and Binding/Unbinding Dynamics from Atomistic Simulations.. *ChemMedChem*, 2011, vol. 6, 1578-1580
- **LIU, K** ; **KOKUBO, H.** Exploring the Stability of Ligand Binding Modes to Proteins by Molecular Dynamics Simulations: A Cross-docking Study. *Journal of Chemical Information and Modeling*, 2017, vol. 57, 2514-2522
- **MOLLICA, L.** ; **DECHERCHI, S.** ; **ZIA, S.R.** ; **GASPARI, R** ; **CAVALLI, A** ; **ROCCHIA, W.** *Kinetics of protein-ligand unbinding via smoothed potential molecular dynamics simulations*, 2015
- **AHINKO, M** ; **NIINIVEHMAS, S** ; **JOKINEN, E.** ; **PENTIKÄINEN, O.T.** Suitability of MMGBSA for the selection of correct ligand binding modes from docking results.. *Chemical Biology & Drug Design*, 2018, vol. 93, 522-538
- **LIU, P.-F** ; **TSAI, K.-L.** ; **HSU, C.-J** ; **TSAI, W.-L.** ; **CHENG, J.-S** ; **CHANG, H.-W** ; **SHIAU, C.-W** ; **GOAN, Y.-G** ; **TSENG, H.-H** ; **WU, C.-H**. Drug Repurposing Screening Identifies Tioconazole as an ATG4 Inhibitor that Suppresses Autophagy and Sensitizes Cancer Cells to Chemotherapy. *Theranostics*, 2018, vol. 8, 830-845
- **SUN, H** ; **LI, Y** ; **TIAN, S** ; **XU, L** ; **HOU, T**. Assessing the performance of MM/PBSA and MM/GBSA methods. 4. Accuracies of MM/PBSA and MM/GBSA methodologies evaluated by various simulation protocols using PDBbind data set.. *Phys. Chem. Chem. Phys.*, 2014, vol. 16, 16719-16729
- **YANG, T** ; **WU, J.C.** ; **YAN, C** ; **WANG, Y** ; **LUO, R** ; **GONZALES, M.B** ; **DALBY, K.N** ; **REN, P**. Virtual screening using molecular simulations. *Proteins: Structure, Function, and Bioinformatics*, 2011, vol. 79, 1940-1951
- **ZHANG, X** ; **PEREZ-SANCHEZ, H** ; **C. LIGHT-STONE, F**. A Comprehensive Docking and MM/GBSA Rescoring Study of Ligand Recognition upon Binding Antithrombin.. *Current Topics in Medicinal Chemistry*, 2017, vol. 17, 1631-1639
- **EASTMAN, P** ; **SWAILS, J** ; **CHODERA, J.D.** ; **MCGIBBON, R.T.** ; **ZHAO, Y** ; **BEAUCHAMP, K.A.** ; **WANG, L.-P** ; **SIMMONETT, A.C** ; **HARRIGAN, M.P** ; **STERN, C.D**. OpenMM 7: Rapid development of high performance algorithms for molecular dynamics. *PLOS Computational Biolog*, 2017, vol. 13
- **CASE, D.A.** ; **BELFON, K** ; **BEN-SHALOM, I.Y** ; **BROZELL, S.R** ; **CERUTTI, D.S** ; **CHEATHAM, T.E** ; **III, CRUZEIRO, VW.D** ; **DARDEN, T.A.** ; **DUKE, R.E** ; **GIAMBASU, G**. AMBER 2020. University of California, 2020
- **MIYAMOTO, S** ; **KOLLMAN, P.A**. Settle: An analytical version of the SHAKE and RATTLE algorithm for rigid water models. *Journal of Computational Chemistry*, 1992, vol. 13, 952-962
- **GENHEDEN, S** ; **RYDE, U.** The MM/PBSA and MM/GBSA methods to estimate ligand-binding affinities.. *Expert Opinion on Drug Discovery*, 2015, vol. 10, 449-461
- **GREENIDGE, P.A.** ; **KRAMER, C** ; **MOZZICONAC-CI, J.-C** ; **WOLF, R.M**. MM/GBSA Binding Energy Prediction on the PDBbind Data Set: Successes, Failures, and Directions for Further Improvement. *Journal of Chemical Information and Modeling*, 2012, vol. 53, 201-209
- **KOLLMAN, P.A.** ; **MASSOVA, I** ; **REYES, C** ; **KUHN, B** ; **HUO, S** ; **CHONG, L** ; **LEE, M** ; **LEE, T** ; **DUAN, Y** ; **WANG, W**. Calculating Structures and Free Energies of Complex Molecules: Combining Molecular Mechanics and Continuum Models. *Accounts of Chemical Research*, 2000, vol. 33, 889-897

- **MASSOVA, I** ; **KOLLMAN, P.A**. Combined molecular mechanical and continuum solvent approach (MM-PBSA/GBSA) to predict ligand binding.. *Perspectives in Drug Discovery and Design*, 2000, vol. 18, 113-135
- **MILLER, B.R** ; **MCGEE, T.D.** ; **SWAILS, J.M** ; **HOMEYER, N** ; **GOHLKE, H** ; **ROITBERG, A.E**. MMPBSA.py: An Efficient Program for End-State Free Energy Calculations. *Journal of Chemical Theory and Computation*, 2012, vol. 8, 3314-3321
- **CASE, D.A.** ; **BELFON, K** ; **BEN-SHALOM, I.Y** ; **BROZELL, S.R** ; **CERUTTI, D.S** ; **CHEATHAM, T.E** ; **III, CRUZEIRO, V.W.D** ; **DARDEN, T.A** ; **DUKE, R.E** ; **GIAMBASU, G**. AMBER 2020. University of California, 2020
- **ROE, D.R** ; **CHEATHAM, T.E**. PTRAJ and CPPTRAJ: Software for Processing and Analysis of Molecular Dynamics Trajectory Data. *Journal of Chemical Theory and Computation*, 2013, vol. 9, 3084-3095
- **GOWERS, R.** ; **LINKE, M** ; **BARNOUD, J** ; **REDDY, T** ; **MELO, M** ; **SEYLER, S** ; **DOMAŃSKI, J.** ; **DOTSON, D** ; **BUCHOUX, S** ; **KENNEY, I**. MDAnalysis: A Python Package for the Rapid Analysis of Molecular Dynamics Simulations. *Proceedings of the 15th Python in Science Conference*, 2016
- **MICHAUD-AGRAWAL, N** ; **DENNING, E.J** ; **WOOLF, T.B** ; **BECKSTEIN, O**. MDAnalysis: A toolkit for the analysis of molecular dynamics simulations. *Journal of Computational Chemistry*, 2011, vol. 32, 2319-2327
- **KOLLMAN, P.A.** ; **MASSOVA, I** ; **REYES, C** ; **KUHN, B** ; **HUO, S** ; **CHONG, L** ; **LEE, M** ; **LEE, T** ; **DUAN, Y** ; **WANG, W**. Calculating Structures and Free Energies of Complex Molecules: Combining Molecular Mechanics and Continuum Models.. *Accounts of Chemical Research*, 2000, vol. 33, 889-897
- **MASSOVA, I** ; **KOLLMAN, P.A**. Combined molecular mechanical and continuum solvent approach (MM-PBSA/GBSA) to predict ligand binding. *Perspectives in Drug Discovery and Design*, 2000, vol. 18, 113-135